# EUROPÄISCHE PATENTSCHRIFT

(11) **EP 1 470 227 B1**
(45) Veröffentlichungstag und Bekanntmachung des Hinweises auf die Patenterteilung: **11.04.2007**
(21) Anmeldenummer: 03704484.9
(22) Anmeldetag: 28.01.2003
(51) Int. Cl.: C12N 15/53, C12N 9/02, C12N 1/21, C12P 33/02, C12N 15/63, C12R 1/06, C12R 1/07, C12R 1/19, C12R 1/38

(54) **Verfahren zur selektiven Einführung einer Doppelbindung in ein Steroidgerüst**
Method for selective introduction of a double bond in a steroid
Procédé d'introduction sélective d'une double liaison dans un stéroide

(30) Priorität: 01.02.2002 DE 10204798
(43) Veröffentlichungstag der Anmeldung: 27.10.2004
(73) Patentinhaber: Bayer Schering Pharma AG, 13353 Berlin (DE)
(72) Erfinder: SPELLIG, Tilman, 10439 Berlin (DE)
(86) Internationale Anmeldenummer: PCT/EP2003/000855
(87) Internationale Veröffentlichungsnummer: WO 2003/064653

(56) Entgegenhaltungen:
- WO-A-91/12326
- US-A- 3 102 080
- MOLNAR I ET AL: "Molecular cloning, expression in Streptomyces lividans, and analysis of a gene cluster from Arthrobacter simplex encoding 3-ketosteroid-delta1-dehydrogenase, 3-ketosteroid-delta 5-isomerase and a hypothetical regulatory protein" MOLECULAR MICROBIOLOGY, BLACKWELL SCIENTIFIC, OXFORD, GB, Bd. 15, Nr. 5, März 1995 (1995-03), Seiten 895-905, XP000926143 ISSN: 0950-382X in der Anmeldung erwähnt -& DATABASE EM_PRO [Online] EMBL; 29. Juni 1995 (1995-06-29) MOLNAR ET AL.: "Arthrobacter simplex ksdR, ksdD and ksdI genes for hypothetic regulatory protein, 3-ketosteroid-1-dehydrogenase and 3-ketosteroid-5-isomerase" retrieved from EBI, accession no. ASKSD Database accession no. D37969 XP002248347 in der Anmeldung erwähnt -& DATABASE SWALL [Online] 1. Februar 1997 (1997-02-01) MOLNAR ET AL.: "3-ketosteroid-1-dehydrogenase (EC 1.3.99.4)" retrieved from EBI Database accession no. P7
- MORII S ET AL: "3-KETOSTEROID-DELTA1-DEHYDROGENASE OF RHODOCOCCUS RHODOCHROUS: SEQUENCING OF THE GENOMIC DNA AND HYPEREXPRESSION, PURIFICATION, AND CHARACTERIZATION OF THE RECOMBINANT ENZYME" JOURNAL OF BIOCHEMISTRY, JAPANESE BIOCHEMICAL SOCIETY, TOKYO, JP, Bd. 124, Nr. 5, November 1998 (1998-11), Seiten 1026-1032, XP000891250 ISSN: 0021-924X -& DATABASE EM_PRO [Online] 14. Oktober 1997 (1997-10-14) MORII ET AL.: "Rhodococcus rhodocrous gene for 3-ketosteroid-delta1-dehydrogenase, complete cds" retrieved from EBI Database accession no. AB007847 XP002248349 in der Anmeldung erwähnt -& DATABASE SWALL [Online] 1. Januar 1998 (1998-01-01) MORII ET AL.: "3-ketosteroid-delta1-dehydrogenase" retrieved from EBI Database accession no. O24819 XP002248350
- DATABASE EM_PRO [Online] EMBL; 2. Mai 2001 (2001-05-02) FLEISCHMANN ET AL.: "Mycobacterium tuberculosis CDC1551, section 252 of 280 of the complete genome" retrieved from EBI Database accession no. AE007166 XP002258868 -& DATABASE SWALL [Online] 1. Februar 1997 (1997-02-01) COLE ET AL.: "Hypothetical 60.6 kDa protein (3-ketosteroid-delta-1-dehydrogenase, putative)" retrieved from EBI Database accession no. P71864 XP002258869 & NATURE, Bd. 393, 1998, Seiten 537-544,

## Beschreibung

Die vorliegende Erfindung betrifft ein Verfahren zur selektiven Einführung einer Doppelbindung in ein Steroidgerüst zur Überexpression von Dehydrogenasen, besonders Δ¹-Dehydrogenasen, insbesondere 3-Ketosteroid-Δ¹-Dehydrogenasen sowie die für die Überexpression verwendeten Plasmide und DNA-Sequenzen.
Die 3-Ketosteroid-Δ¹-Dehydrogenase ist ein Enzym, welches im Steroidstoffwechsel eine wichtige Funktion erfüllt. Mit Hilfe dieses Enzyms wird die selektive Einführung einer Doppelbildung an Position 1 im Steroidgerüst ermöglicht. Diese Reaktion ist für die Synthese einer Vielzahl von Pharma-Wirkstoffen [z.B. Betamethason, Deflazacort, Fluocortolon, Hydroxysäure, Prednisolon, etc.] von großer Bedeutung. Es wäre wünschenswert, große Mengen dieses Enzyms für eine mikrobiologische Umsetzung verfügbar zu machen.
Für Verfahren zur mikrobiellen Stoffumwandlung, wie z.B. Steroidtransformationen, werden in der Regel Wildstämme von Hefen, Pilzen und Bakterien eingesetzt [s. u.a. Kieslich K (1980), *Steroid conversions,* In: *Economic Microbiology - Microbial Enzymes and Transformation,* Rose AH (ed), Academic Press, London, Vol. V, pp 370-453; Kieslich K. and Sebek OK (1980) *Microbal transformations of steroids,* In: *Annual Reports on Fermentation Processes,* Perlman D (ed), Academic Press, New York, Vol. 3, pp 275-304; Kieslich K (ed) (1984) *Biotransformation, Biotechnology,* Vol. 6a, Rehm HJ and Reed G (eds), Verlag Chemie, Weinheim]. Vereinzelt werden auch von Wildstämmen abgeleitete, durch klassische Mutagenese und Selektionsverfahren gewonnene Mutanten verwendet [s. u.a. U.S. Pat. 3,102,080; Seidel L and Hörhold C (1992) J Basic Microbiol 32:49-55; EP 0322081 B1; U.S. Pat. 5,298,398]. So wird z. B. bei biotechnologischen Verfahren zur selektiven Dehydrierung die endogene katalytische Aktivität verschiedener Mikroorganismen, u.a. *Arthrobacter simplex* und *Bacillus sphaericus,* genutzt [Sedlaczek (1988) Crit Rev Biotechnol. 7:187-236; U.S. Pat. 2,837,464; U.S. Pat. 3,010,876; U.S. Pat. 3,102,080].
Es ist ferner bekannt, daß Δ¹-Dehydrogenasegene von *Arthrobacter simplex* [Choi KP et al. (1995) *J Biochem* **117**:1043-1049; Molnar 1 et al. (1995) *Mol Microbiol* **15**:895-905], *Comamonas testosteroni* [Plesiat P et al. (1991) *J Bacteriol* **173**:7219-7227] und *Nocardia* opaca [Drobnic K et al. (1993) *Biochem Biophys Res Com* **190**:509-515; SUISS-PROT AC: Q04616] kloniert, sequenziert und funktionell charakterisiert wurden. Auch von *Mycobacterium tuberculosis* und *Rhodococcus rhodochrous* wurden DNA-Sequenzen publiziert, die wegen ihrer Ähnlichkeit zu den oben genannten Δ¹-Dehydrogenasegenen als mutmaßliche Dehydrogenasegene betrachtet werden können [http://www.sanger.ac.uk/Projects/M_tuberculosis; GenBank AB007847].
Eine Beschränkung der bekannten Biotransformations-Verfahren liegt darin, daß diese im allgemeinen Prozeßoptimierungen sind, die sich überwiegend auf die Verbesserung der Reaktionsbedingungen und Verfahrensparameter konzentrieren, wie z.B. Art und Zusammensetzung der Nährstoffe, Verfahrensführung, Substratapplikation, etc. Insbesondere die Verfahren zur selektiven Dehydrierung haben eine Reihe von Nachteilen wie z.B. i) vollständige Umsetzung des Edukts nur bei sehr niedrigen Substratkonzentrationen [U.S. Pat. 3,102,080], ii) lange Laufzeiten und iii) die Bildung von Nebenzonen - wie z. B. 11α-Hydroxyandrosta-1,4-dien-3,17-dion bei der Umsetzung von Hydrocortison zu Prednisolon, welche durch aufwendige Reinigungsverfahren abgetrennt werden müssen. Diese Nachteile führen dazu, daß der Herstellungsprozeß sehr aufwendig ist.

Es wurde nun gefunden, daß durch gezielte Veränderung der die Stoffumwandlung katalysierenden Mikroorganismen mit molekularbiologischen Methoden bessere, effizientere und zielgerichtetere Biotransformationen von Steroidmolekülen erzielt werden können. Die Biotransformationsreaktionen werden mit Bakterien durchgeführt, die Plasmide zur Überexpression von 3-Ketosteroid-A'-Dehydrogenasegenen enthalten.
Zu den verwendeten Bakterien gehören insbesondere Vertreter der gram-positiven Gattung *Bacillus,* wie *Bacillus subtilis, Bacillus sphaericus, Bacillus licheniformis, Bacillus lentus* und *Bacillus megaterium,* aber auch *gram*-negative Vertreter, wie *Escherichia coli* und *Pseudomonas species.*
Durch gezielte Stammentwicklung mit molekularbiologischen Methoden werden Mikroorganismen konstruiert, die die Synthese der Wirkstoffe beschleunigen und vereinfachen, indem i) der Einsatz sehr hoher Substratkonzentrationen bei ii) unveränderter Laufzeit möglich wird, ohne daß iii) störende Nebenzonen entstehen.
Insbesondere wird hier die selektive Dehydrierung an Position 1 des Steroidgerüsts beschrieben, wobei aus *Bacillus sphaericus* isolierte 3-Ketosteroid-Δ¹-Dehydrogenasegene eingesetzt werden.
Erfindungsgemäß wird nun ein Verfahren zur selektiven Einführung einer Doppelbindung in ein Steroidgerüst durch Überexpression von Dehydrogenasen beschrieben, das dadurch gekennzeichnet ist, daß man
a) ein Dehydrogenasegen aus einem Bakterium *Bacillus sphaericus* isoliert, kloniert und amplifiziert,
b) Promotor- und Terminator-Elemente des Dehydrogenasegens oder andere Promotor- und Terminator-Elemente aus dem gleichen oder einem weiteren Bakterium isoliert, kloniert und amplifiziert,
c) Expressionsplasmide konstruiert, worin das Dehydrogenasegen aus a), flankiert von Promotor-und Terminatorsequenz des Dehydrogenasegens oder von anderen Promotor- und Terminator-Elementen aus b), enthalten ist,
d) mit dem unter c) hergestellten Expressionsplasmid Bakterien transformiert und
e) die so hergestellten Bakterien kultiviert und mit diesen Kulturen die selektive Dehydrierung im Steroidgerüst durchführt, wobei
   i) eine hohe Substratkonzentration bei unveränderter Laufzeit eingesetzt wird und
   ii) keine störenden Nebenzonen entstehen.

Besonders betrifft die vorliegende Erfindung ein Verfahren zur selektiven Einführung einer Doppelbindung in ein Steroidgerüst durch Überexpression von Δ¹-Dehydrogenasen, das dadurch gekennzeichnet ist, daß man
a) ein Δ¹-Dehydrogenasegen aus einem Bakterium *Bacillus sphaericus* isoliert, kloniert und amplifiziert,
b) Promotor- und Terminator-Elemente des Δ¹-Dehydrogenasegens oder andere Promotor- und Terminator-Elemente aus dem gleichen oder einem weiteren Bakterium isoliert, kloniert und amplifiziert,
c) Expressionsplasmide konstruiert, worin das Δ¹-Dehydrogenasegen aus a), flankiert von Promotor-und Terminatorsequenz des Δ¹-Dehydrogenasegens oder von anderen Promotor- und Terminator-Elementen aus b), enthalten ist,
d) mit dem unter c) hergestellten Expressionsplasmid Bakterien transformiert und
e) die so hergestellten Bakterien kultiviert und mit diesen Kulturen die selektive Dehydrierung im Steroidgerüst durchführt, wobei
   i) eine hohe Substratkonzentration bei unveränderter Laufzeit eingesetzt wird und
   ii) keine störenden Nebenzonen entstehen.

Insbesondere betrifft die vorliegende Erfindung ein Verfahren zur selektiven Einführung einer Doppelbindung in ein Steroidgerüst durch Überexpression von 3-Ketosteroid-Δ¹-Dehydrogenasen, das dadurch gekennzeichnet ist, daß man
a) das 3-Ketosteroid-Δ¹-Dehydrogenasegen aus einem Bakterium *Bacillus sphaericus* isoliert, kloniert und amplifiziert
b) Promotor- und Terminator-Elemente des 3-Ketosteroid-Δ¹-Dehydrogenasegens oder andere Promotor- und Terminator-Elemente aus dem gleichen oder einem weiteren Bakterium isoliert, kloniert und amplifiziert,
c) Expressionsplasmide konstruiert, worin das 3-Ketosteroid-Δ¹-Dehydrogenasegen aus a), flankiert von Promotor-und Terminatorsequenz des 3-Ketosteroid-Δ¹-Dehydrogenasegens oder von anderen Promotor- und Terminator-Elementen aus b), enthalten ist,
d) mit dem unter c) hergestellten Expressionsplasmid Bakterien transformiert und
e) die so hergestellten Bakterien kultiviert und mit diesen Kulturen die selektive Dehydrierung an Position 1 im Steroidgerüst durchführt, wobei
   i) eine hohe Substratkonzentration bei unveränderter Laufzeit eingesetzt wird und
   ii) keine störenden Nebenzonen entstehen.

Die in den Verfahrensschritten b) und d) genannten Bakterien können zur *gram-*positiven Gattung *Bacillus,* wie *Bacillus spec., Bacillus subtilis, Bacillus sphaericus, Bacillus megaterium, Bacillus licheniformis, Bacillus lentus* sowie zu den den *gram-*positiven Vertretern *Arthrobacter simplex* und *Brevibacterium maris* bzw. zu den *gram*-negativen Vertretern *Escherichia coli* und *Pseudomonas species* gehören. Die vorliegende Erfindung betrifft insbesondere das 3-Ketosteroid-Δ¹-Dehydrogenase-Gen aus *Bacillus sphaericus* mit Promotor- und Terminator-Elementen gemäß Seq. ID No. 9 bzw. Seq. ID No. 10 und gemäß Seq. ID No. 11.

Zum Einbringen der erfinderischen DNA-Sequenzen in die Wirtszellen werden Plasmide verwendet, die mindestens eine der oben genannten DNA-Sequenzen enthalten. Auf den Plasmiden werden die Δ¹-Dehydrogenasegene mit geeigneten Promotoren und Terminatoren versehen, die zur Überexpression in Bakterien erforderlich sind. Geeignete Promotoren und Terminatoren sind z.B. Promotor und Terminator des 3-Ketosteroid-Δ¹-Dehydrogenasegens von *Bacillus sphaericus* gemäß Seq. ID No. 9, konstitutive Promotoren wie p*(veg)* oder Promotoren der Bacteriophagen Φ29 und SPO1, induzierbare Promotoren wie p*(aprE)* oder p(*sacB*) aus *Bacillus subtilis,* Hybridpromotoren wie z.B. ein *lacl* kontrollierter SPO1-Promotor, Terminatoren von *Escherichia coli* wie *t(rrnB)* oder von *Bacillus subtilis* wie *t(senS)* oder *t(senN)* [s. u.a. Doi RH (1984) In: Biotechnology and Genetic Engineering Reviews, Vol 2, Russell GE (ed), Intercept, Newcastle Upon Tyne, UK, pp 121-153; Le Grice SFJ et al. (1986) In: *Bacillus Molecular Genetics and Biotechnology Applications,* Ganesan AT and Hoch JA (eds), Academic Press, New York, 433-445; Mountain A (1989) In: *Bacillus,* Harwood CR (ed), Plenum Press, New York, pp 73-114; Le Grice SFJ (1990) *Meth Enzymol* 185:210-214; Wang and Doi (1992) In: *Biology of Bacilli: Applications to lndustry,* Doi et al. (eds), Massachusetts, Butterworth-Heinemann, pp 143-188].

Die Plasmide sind ebenfalls Gegenstand der vorliegenden Erfindung.

Die Plasmide können zur Transformation von Bakterien, die zur Überexpression von Δ¹-Dehydrogenasen befähigt sind, verwendet werden.
Die Erfindung betrifft auch die DNA-Sequenzen mit 3-Ketosteroid-Δ¹-Dehydrogenase-Aktivität, deren DNA-Sequenzen eine Homologie von mehr als 80%, insbesondere eine Homologie von mehr als 90% und vorzugsweise eine Homologie von mehr als 95% im Vergleich zum Seq. ID No. 10 aufweisen.
Die Erfindung betrifft auch Protein-Sequenzen mit 3-Ketosteroid-Δ¹-Dehydrogenase-Aktivität, die eine Homologie von mindestens 90%, insbesondere eine Homologie von mindestens 95% im Vergleich zum Seq. ID No. 11 aufweisen.
Die Erfindung betrifft auch Promotoren, insbesondere den 3-Ketosteroid-Δ¹-Dehydrogenase-Promotor aus *Bacillus sphaericus* mit der DNA-Sequenz Seq. ID. No. 9, sowie homologe Promotoren, die eine Homologie zur Seq. ID No. 9 von mehr als 80%, vorzugsweise mehr als 90% und besonders bevorzugt mehr als 95% im Vergleich zum Seq. ID No. 9 aufweisen.
Die Erfindung betrifft ferner die *Bacillus sphaericus* 3-Ketosteroid-Δ¹-Dehydrogenase Oligonukleotide gemäß den Sequenzen Seq. ID No. 15, Seq. ID No. 16, Seq. ID No.17 und Seq. ID No. 18, und die parS Oligonukleotide gemäß den Sequenzen Seq. ID No. 19 und Seq. ID No. 20.

Die erfindungsgemäßen DNA-Sequenzen und Proteine können zur selektiven Dehydrierung von Steroiden eingesetzt werden.

Dehydrierte Steroide sind z.B. Betamethason, Clobetason, Clocortolon, Δ¹-11β, 17α-Dihydroxy-6α,9α-difluor-16α-methylprogesteron, Deflazacort, Dexamethason, Diflocortolon, Fluocinolonacetonid, Fluocortolon, Hydroxysäure und Prednisolon und Derivate der genannten Verbindungen.

### Hinterlegungen

Die in der Anmeldung genannten Bakterien-Stämme können über die jeweiligen Hinterlegungsstellen bezogen werden, z.B. von der DSM ⇒ Deutsche Sammlung von Mikroorganismen und Zellkulturen GmbH, Mascheroder Weg 1b, D-38124 Braunschweig; ATCC ⇒ American Type Culture Collection, Rockville, Maryland, USA; NRRL ⇒ Northern Utilization Tesearch and Development Division, Peoria, Illinois, USA; etc.

Zum besseren Verständnis der dieser Erfindung zugrunde liegenden Erfindung werden zunächst die verwendeten Methoden beschrieben.

### 1. Restriktion

Restriktionen von Plasmid- und genomischer DNA wurden - in Abhängigkeit von der Menge eingesetzter DNA [1 bis 20 µg] - in Volumina von 15 bis 100 µl durchgeführt. Die Enzymkonzentration betrug 1 bis 5 Units Restriktionsenzym pro µg DNA. Die Reaktion wurde in einem Puffer durchgeführt, für eine bis drei Stunden inkubiert und im Anschluß auf einem Agarosegel analysiert [Sambrook et al. (1989) *Molecular Cloning: a laboratory manual,* Cold Spring Harbor Laboratory Press, Cold Spring Harbor, New York].

### 2. Agarose-Gelelektrophorese

Gelelektrophoresen wurden in Minigel- [BioRad], Midi-Wdegel- [Biometra] und Maxigelapparaturen [Biometra] durchgeführt. Es wurden je nach Trennproblem Agarosegele mit 0,8 % bis 4 % [w/v] Agarose in 0,5 x TBE-Puffer verwendet. Die Elektrophorese erfolgte mit 0,5 x TBE als Laufpuffer. DNA-Fragmente wurden mit Ethidiumbromid gefärbt und auf einem Transilluminator sichtbar gemacht [Sambrook et al. (1989) *Molecular Cloning: a laboratory manual,* Cold Spring Harbor Laboratory Press, Cold Spring Harbor, New York].

### 3. Elution von DNA aus einem Agarosegel

Präparative Restriktionsansätze wurden im Agarosegel nach Größe getrennt. Die gewünschten Banden wurden mit einem Skalpell ausgeschnitten. Das zu isolierende DNA-Fragment wurde mit Hilfe des "Jetsorb Kit" [Genomed] unter Berücksichtigung der Vorschrift des Herstellers wiedergewonnen und in TE-Puffer aufgenommen.

### 4. Phosphorylierung von Oligonukleotiden

50 pmol Oligonucleotid wurden im vom Hersteller empfohlenen Puffer in Anwesenheit von 0.1 mM ATP und 20 Units T4 Polynucleotidkinase 45 min bei 37 °C inkubiert. Eine Enzyminaktivierung erfolgte bei 68 °C [Sambrook et al. (1989) *Molecular Cloning: a laboratory manual,* Cold Spring Harbor Laboratory Press, Cold Spring Harbor, New York].

### 5. Ligation

Zur Ligation wurden geeignete Mengen dephosphorylierter, linearisierter Vektor-DNA und und Fragment-DNA in einem molaren Verhältnis von 1:5 eingesetzt Die Reaktion erfolgte in einem Volumen von 10 µl mit 1 Unit T4-DNA-Ligase im vom Hersteller empfohlenen Puffer bei 16°C über Nacht im Wasserbad [Sambrook et al. (1989) *Molecular Cloning:* a *laboratory manual,* Cold Spring Harbor Laboratory Press, Cold Spring Harbor, New York].

### 6. Transformation von Escherichia coli

Kompetente *E. coli* Zellen wurden durch CaCl₂ - Behandlung erhalten und bei -80°C gelagert. In der Regel wurde ein 10 µl Ligationsansatz mit 200 µl kompetenten Zellen inkubiert. Die Transformationsansätze wurden auf LB-Agar mit dem jeweils erforderlichen Antibiotikumzusatz plattiert und 16 Stunden bei 37°C bebrütet. Eine Herstellung kompetenter Zellen und eine Transformation erfolgten nach Sambrook et al. (1989) *Molecular Cloning: a laboratory manual,* Cold Spring Harbor Laboratory Press, Cold Spring Harbor, New York.

### 7. Transformation von Bacillus subtilis

Die Transformation von *Bacillus subtilis* erfolgte nach dem von Cutting SM und Vander Horn PB beschriebenen Zwei-Stufen-Verfahren [In: *Molecular Biological Methods for Bacillus* (1990), Harwood CR and Cutting SM (eds), John Wiley & Sons, Chichester].

### 8. Transformation von Bacillus sphaericus

*Bacillus sphaericus* wurde in Anlehnung an ein von Taylor und Burke (1990) publiziertes Verfahren durch Elektroporation transformiert [FEMS Microbiol Lett 66:125-128]. Die Zellen wurden in MM2G-Medium [0.3 % (w/v) Fleischextrakt, 0.8 % (w/v) Hefeextrakt, 1 % (w/v) Pepton, 0.2 % (w/v) Glucose, 0.7 % (w/v) NaCl, 7.36 g/L K₂HPO₄, 2.65 g/L KH₂PO₄, 5 ml/L 100 % Glycerin, pH 7] über Nacht angezogen, 1:20 in frisches MM2G-Medium transferiert und 90 min bei 37 °C und 250 rpm kultiviert. Die Zellen wurden pelletiert, 3 x mit 10 % Glycerin gewaschen und anschließend in 750 µl Glycerin aufgenommen. 50 µl Zellsuspension wurden in einer Elektroporationsküvette mit Plasmid-DNA gemischt, auf Eis inkubiert und in das Elektroporationsgerät [Biorad Gene Pulser^{™}] gestellt [2.5 kV, 25 µF, 600 Ω]. Die Zellen wurden zur Regeneration 90 min bei 30 °C in MM2G-Medium inkubiert und im Anschluß auf TBAB-Agar/5 µg Neomycin [Tryptose Blood Agar Base (Difco)] plattiert und 24 h bei 30 °C bebrütet.

### 9. Plasmidminipräparation aus Escherichia coli

Minipräparationen wurden nach dem Prinzip der alkalischen Lyse [Sambrook et al. (1989) *Molecular Cloning:* a *laboratory manual,* Cold Spring Harbor Laboratory Press, Cold Spring Harbor, New York] durchgeführt. Einzelkolonien wurden in Reagenzgläsern mit 4 ml LB-Medium und Selektion über Nacht angezogen. 2 ml davon wurden zur Präparation eingesetzt.

### 10. Plasmidminipräparation aus Bacillus subtilis und Bacillus sphaericus

Die Präparation von Plasmiden aus *Bacillus subtilis* und *Bacillus sphaericus* erfolgte über Säulen der Firma Genomed ["Jetstar Kit Mini"] nach dem vom Hersteller vorgegebenem Protokoll. Um eine vollständige Lyse der Zellen zu gewährleisten wurde das in Puffer E1 aufgenommene Zellpellet mit 5 mg/ml Lysozym versetzt und die Zellen für eine Stunde bei 37°C inkubiert.

### 11. Plasmidmaxipräparation aus Escherichia coli, Bacillus subtilis und Bacillus sphaericus

Plasmid-Maxipräparation wurde mit dem "Jetstar Kit Maxi" der Firma Genomed durchgeführt. Die Stämme wurden in 200 ml LB-Medium in Anwesenheit eines Antibiotikums über Nacht angezogen. Die Präparation der Plasmide erfolgte nach dem vom Hersteller angegebenem Protokoll. Um eine vollständige Lyse von *Bacillus subtilis* und *Bacillus sphaericus* zu gewährleisten, wurden die in Puffer E1 aufgenommenen Zellpellets zusätzlich mit 5 mg/ml Lysozym versetzt und die Zellen für eine Stunde bei 37°C inkubiert.

### 12. Präparation genomischer DNA aus Bacillus species

200 ml einer dichtgewachsenen Bakterienkultur wurden pelletiert und in 11 ml Lösung I [50 mM Tris-HCl pH 8, 50 mM EDTA, 1 % (v/v) Triton x-100, 200 µg/ml Rnase] suspendiert. Die Suspension wurde mit Lysozym [5 mg/ml] und 500 µl Proteinase K [20 mg/ml] versetzt und > 30 min bei 37 °C inkubiert. Im Anschluß daran wurden 4 ml Lösung II [3 M Guanidinium-Hydrochlorid, 20 % (v/v) Tween] hinzugegeben und der Ansatz für 30 min bei 50°C inkubiert. Nicht aufgelöste Partikel wurden pelletiert und verworfen. Die im Lysat gelöste chromosomale DNA wurde per Anionen-Austauschchromatographie ["Jetstar Kit Maxi" der Firma Genomed, s. das vom Hersteller angegebene Protokoll] gereinigt.

### 13. Polymerase Ketten Reaktion

Die Reaktionsbedingungen für die PCR wurden für jeden Einzelfall optimiert. In der Regel wurden 0,1 bis 0,5 µg Template - DNA, 10 mM dNTPs, je 50 pmol 5'-und 3'-Primer sowie 2,5 Units Pwo-Polymerase [Boehringer Mannheim] im vom Hersteller empfohlenen Puffer in 100 µl Gesamtvolumen vereinigt. Je nach Template - DNA wurden dem Ansatz bis zu 10 % DMSO zugesetzt. Die PCR wurde in einem "Biometra Trio Thermoblock" durchgeführt. Das Temperaturprofil wurde für jede Fragestellung neu angepaßt. Die Annealing-Temperatur variierte zwischen 50°C [wenig stringente Bedingungen] und 65 °C. [s. PCR 1: A *Practical Approach,* McPherson et al. (eds), Oxford University Press (1991)]

### 14. Southern - Analysen

Im Agarosegel nach Größe getrennte DNA wurde nach dem Kapillar-Blot-Verfahren [Sambrook et al. (1989) *Molecular Cloning: a laboratory manual,* Cold Spring Harbor Laboratory Press, Cold Spring Harbor, New York] auf positiv geladene Nylonmembranen transferiert und durch UV-Bestrahlung kovalent mit der Membran verknüpft.
Hybridisierungen wurden mit Digoxigenin markierten Sonden durchgeführt. Die Markierung der Sonden erfolgte mit dem "DIG - High - Prime" oder dem "PCR DIG Probe Synthesis Kit" von Boehringer Mannheim nach dem vom Hersteller empfohlenen Protokoll.
Zur Hybridisierung wurde ein SDS-Phosphat-Puffer verwendet [7 % SDS (w/v); 0,5 M NaPhosphat pH 7,0]. Je nach Fragestellung wurden stringente oder wenig stringente Hybridisierungsbedingungen gewählt [Sambrook et al. (1989) *Molecular Cloning: a laboratory manual,* Cold Spring Harbor Laboratory Press, Cold Spring Harbor, New York]
Die Detektion gebundener DNA erfolgte mit einem Chemilumiszens-Reagenz [CSPD^{®}] von Boehringer Mannheim nach der vom Hersteller empfohlenen Vorschrift.

### 14. Kolonie-Hybridisierung

Die Übertragung von Kolonien auf Pall BIODYNE^{®} A Membranen [1,2 µm und 0,2 µm Porengröße] wurde nach dem vom Hersteller empfohlenen Verfahren durchgeführt.
Die Hybridisierung erfolgte mit Digoxigenin-markierten Sonden im o.a. SDS-Phosphat-Puffer, die Detektion mit einem Chemilumineszens-Reagens CSPD^{®} von Boehringer Mannheim ["Pall Bio Support" Anwendungs-Information SD1359G].

### 15. DNA-Sequenzanalyse

DNA-Sequenzanalysen erfolgten mit dem GATC^{®} 1500-System. Die Sequenzreaktionen wurden mit dem GATC^{®}-BioCycle Sequencing Kit nach dem vom Hersteller empfohlenen Protokoll durchgeführt und auf einem 4% Polyacrylamid-Harnstoff-Gel analysiert [GATC^{®} 1500-System Protokoll]. Die Detektion erfolgte mit CSPD^{®} [GATC^{®}-Biocyde Sequencing Kit Protokoll].

### 16. Hydrocortison/Hydrocortison-17-acetat → Prednisolon: Aufarbeitung und Analytik

Die Kulturbrühe wurde mit dem 3-fachen Volumen Methanol/ 1 % Essigsäure verdünnt, Ultraschall-behandelt und abgeschleudert. Der Überstand wurde auf einer ODS-Hypersil-Säule [250 x 4.6 mm] mit einem Acetonitril-Wasser-Gradienten bei einer Flußrate von 1 ml/min chromatographiert.
Reihenfolge der Eluenten: Hydrocortison, Prednisolon, 11β-Hydroxyandrosta-1,4-dien-3,17-dion, Hydrocortison-17-acetat, Hydrocortison-21-acetat, Prednisolon-21-acetat.

### 17. 4-Androsten-3,17-dion → Androsta-1,4-dien-3,17-dion: Aufarbeitung und Analytik

Isobutylmethylketon-Extrakte der Kulturbrühe wurden gaschromatographisch analysiert:
Säule 1: 50 m x 0.25 mm, Chrompack WCOT CP5 CB, Filmdicke 0.4 µm
Säule 2: 30 m x 0.25 mm, hp 1701, Filmdicke 0.4 µm
Detektor: FID
Trägergas: Wasserstoff
Säulenvordruck: 175 kPa
Reihenfolge der Eluenten: 4-Androsten-3,17-dion, Androsta-1,4-dien-3,17-dion

### 18. Fluocortolon A Acetat → Fluocortolon: Aufarbeitung und Analytik

Die Kulturbrühe wurde mit Essigsäure auf pH 4-6 eingestellt und anschließend mit dem 4-fachen Volumen Isobutylmethylketon extrahiert. Der Extrakt wurde eingedampft, in Chloroform aufgenommen und auf einer Kromasil 100 Säule [250 x 4 mm] mit einem isokratischen Gradienten von Chloroform:Isooctan:1,4-Dioxan:Ethanol:Wasser 1000:100:50:10:2 bei einer Flußrate von 1.2 ml/min chromatographiert.
Reihenfolge der Eluenten: Fluocortolon A Acetat, Fluocortolon A, Fluocortolon

### 19. 11β,17α-Dihydroxy-6α,9α-difluor-16α-methylprogesteron → Δ¹-11β,17α-Dihydroxy-6α,9α-difluor-16α-methylprogesteron: Aufarbeitung und Analytik

Die Kulturbrühe wurde mit dem 3-fachen Volumen Methanol / 1 % Essigsäure verdünnt, Ultraschall-behandelt und abgeschleudert. Der Überstand wurde auf einer ODS-Hypersil-Säule [250 x 4.6 mm] mit einem Acetonitril-Wasser-Gradienten bei einer Flußrate von 1 ml/min chromatographiert.
Reihenfolge der Eluenten: 11β,17α-Dihydroxy-6α,9α-difluor-16α-methylprogesteron, Δ¹-11β, 17α-Dihydroxy-6α,9α-difluor-16α-methylprogesteron

### 20. 11β,21-Dihydroxy-2'-methyl-5'βH-pregn-4-eno[17,16-d]oxazol-3,20-dion → 11β,21-Dihydroxy-2'-methyl-5'βH-pregna-1,4-dieno[17,16-d]oxazol-3,20-dion (Deflazacortalkohol): Aufarbeitung und Analytik

Die Kulturbrühe wurde turraxiert und anschließend mit dem 4-fachen Volumen Methylisobutylketon extrahiert. Der Extrakt wurde bis zur Trockne eingedampft und im gleichen Volumen Chloroform aufgenommen. Die Probe wurde auf eine Kromasil-100 Säule [250 x 4.6 mm] aufgetragen und mit Diisopropylether : Dichlorethan : 1,4-Dioxan : H₂O (250:150:75:4) bei einer Flußrate von 2 ml/min chromatographiert. Reihenfolge der Eluenten: 11β,21-Dihydroxy-2'-methyl-5'βH-pregn-4-eno[17,16-d]oxazol-3,20-dion, 11β,21-Dihydroxy-2'-methyl-5'βH-pregna-1,4-dieno[17,16-d]oxazol-3,20-dion (Deflazacortalkohol)

### Beschreibung der Abbildungen

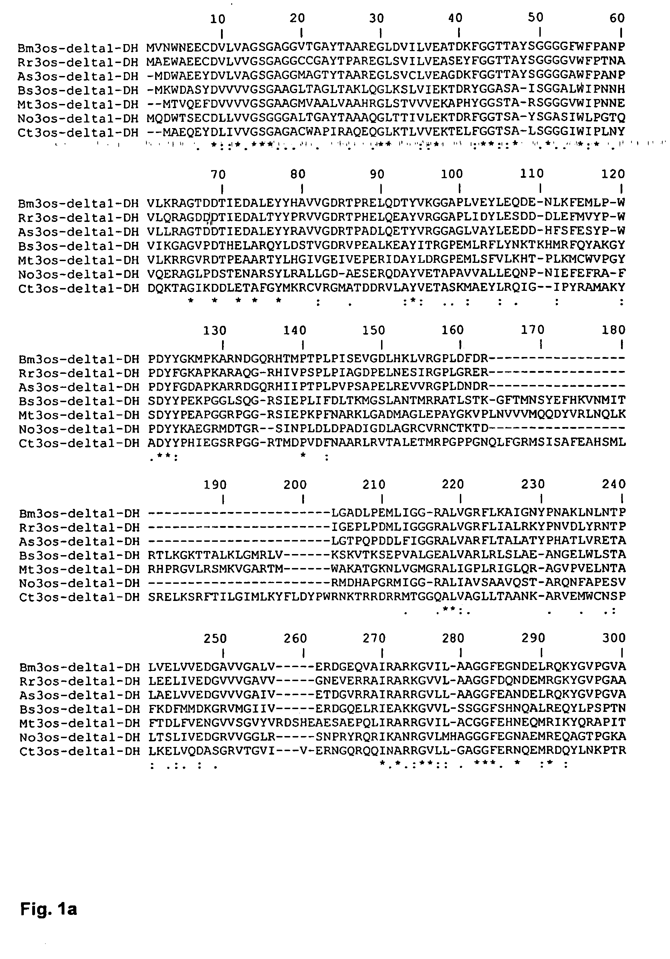

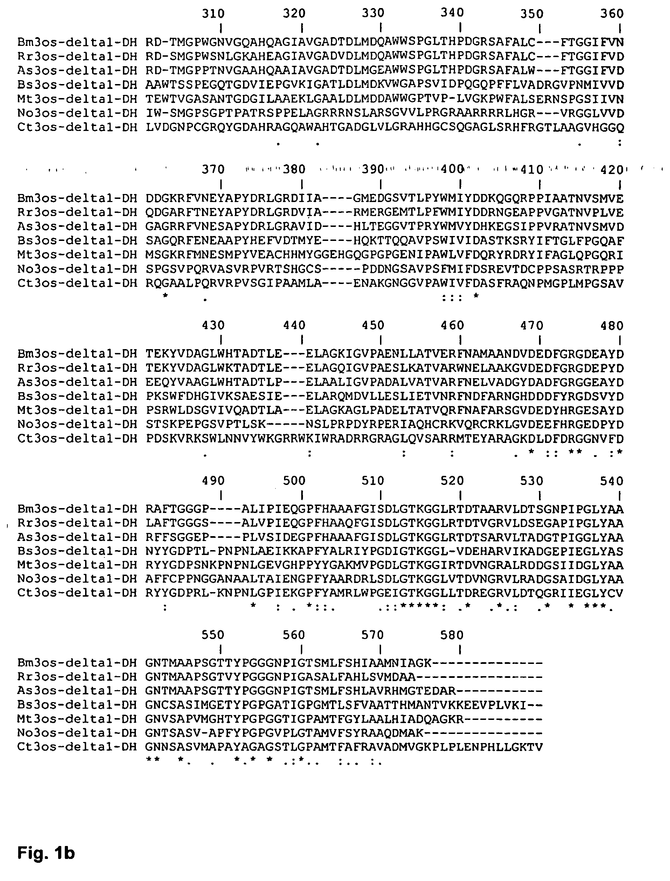

- **Fig. 1a/1b**: zeigt das Alignment aller bekannten 3-Ketosteroid-A'-Dehydrogenasen [CLUSTAL W Algorithmus, Thompson JD et al. (1994) *Nucleic Acids* Res 22:4673-4680].
In der Abbildung bedeuten:
*Bm*3os-delta1-DH = *Brevibacterium maris* 3-Oxosteroid-Δ¹-Dehydrog,enase
*Rr*3os-delta1-DH *= Rhodococcus rhodochrous* 3-Oxosteroid-Δ¹-Dehydrogenase
*As*3os-delta1-DH *= Arthrobacter simplex* 3-Oxosteroid-Δ¹-Dehydrogenase
*Bs*3os-delta1-DH *= Bacillus sphaericus* 3-Oxosteroid-Δ¹-Dehydrogenase
*Mt*3os-delta1-DH = *Mycobacterium tuberculosis* 3-Oxosteroid-Δ¹-Dehydrogenase
*No*3os-delta1-DH *= Nocardia* opaca 3-Oxosteroid-Δ¹-Dehydrogenase
*Ct*3os-delta1-DH = *Comamonas testosteroni* 3-Oxosteroid-Δ¹-Dehydrogenase
Number of perfect matches * 61 ⇒ 10.34 %
Number of high similarity : 48 ⇒ 8.14 %
Number of low similarity . 54 ⇒ 9.15 %
*Bm*3os-delta1-DH [diese Arbeit]; *Rr*3os-delta1-DH [GenBank AC: AB007847]; *As*3os-delta1-DH [Molnar I et al. (1995) *Mol Microbiol* **15**:895-905; GenBank AC: D37969]; Bs3os-delta1-DH [diese Arbeit];
*Mt*3os-delta1-DH [Cosmid Z82098. complement 16520...18211;
http://www.sanger.ac.uk/M_tuberculosis]; *N*o3os-delta1-DH [Drobnic K et al. (1993) *Biochem Biophys Res* Comm **190**:509-515; SUISS-PROT AC: Q04616]; C*t*3os-delta1-DH [Plesiat P et. (1991) *J Bacteriol* **173**:7219-7227; SUISS-PROT AC: Q06401].
- **Fig. 2**: zeigt das Expressionsplasmid TS#196

- **Fig. 3**: zeigt die Umsetzung von EAF/MAF/F zu Pln [1 g/L]: Vergleich Stamm AD#67 mit *Bacillus sphaericus* ATCC 13805 In der Abbildung bedeuten:
EAF ⇒ Hydrocortison-21-acetat
MAF ⇒ Hydrocortison-17-acetat
F ⇒ Hydrocortison
Pln ⇒ Prednisolon
- **Fig. 4**: zeigt die Umsetzung von EAF/MAF/F zu Pln [10 g/L]: Vergleich Stamm AD#67 mit *Bacillus sphaericus* ATCC 13805
In der Abbildung bedeuten:
EAF ⇒ Hydrocortison-21-acetat
MAP ⇒ Hydrocortison-17-acetat
F ⇒ Hydrocortison
Pln ⇒ Prednisolon
- **Fig. 5**: zeigt die Umsetzung von AD zu ADD [1 g/L]: Vergleich Stamm AD#67 mit *Bacillus sphaericus* ATCC 13805
In der Abbildung bedeuten:
AD ⇒ 4-Androsten-3,17-dion
ADD ⇒ Androsta-1 ,4-dien-3,17-dion
- **Fig. 6**: zeigt zeigt die Umsetzung von FCAA zu FC [1 g/L]: Vergleich Stamm AD#116 mit *Bacillus sphaericus* ATCC 13805
In der Abbildung bedeuten:
FCAA ⇒ Fluocortolon A Acetat
FCA ⇒ Fluocortolon A
FC ⇒ Fluocortolon
- **Fig. 7**: zeigt die Umsetzung von DDFMP zu Δ¹-DDFMP [0.2 g/L]: Vergleich Stamm AD#116 mit *Bacillus sphaericus* ATCC 13805
In der Abbildung bedeuten:
DDFMP ⇒ 11 β,17α-Dihydroxy-6α,9α-difluor-16α-methylprogesteron
Δ¹-DDFMP ⇒ Δ¹-11β,17α-Dihydroxy-6α,9α-difluor-16α-methylprogesteron
- **Fig. 8**: zeigt die Umwandlung von EAF/MAF/F zu Pln im 10 L Fermenter [20 g/L]
Vergleich Stamm AD#67 / *Bacillus sphaericus* ATCC 13805
Bedeutung der Abkürzungen siehe oben.
- **Fig. 9**: zeigt die Umsetzung von 11β,21-Dihydroxy-2'-methyl-5'βH-pregn-4, eno[17,16-d]oxazol-3,20-dion zu 11β,21-Dihydroxy-2'-methyl-5`βH-pregna-1,4-dieno[17,16-d]oxazol-3,20-dion (Deflazacortalkohol) [1 g/L]: Vergleich Stamm AO#205 mit *Bacillus sphaericus* ATCC 13805

Die nachfolgenden Klonierungs, Isolierungs- und Kontruktions-Beispiele beschreiben die biologische Ausführbarkeit der Erfindung, ohne diese auf die Beispiele zu beschränken.

### Beispiel 1

### Klonierung des 3-Ketosteroid-Δ¹-Dehydrogenasegens aus Bacillus sphaericus ATCC 13805

Zur Isolierung des 3-Ketosteroid-Δ¹-Dehydrogenasegens aus *Bacillus sphaericus* ATCC 13805 wurde unter Verwendung degenerierter Primer in einer PCR-Reaktion eine homologe Sonde aus genomischer DNA von *Bacillus sphaericus* isoliert: Unter wenig stringenten Bedingungen wurde mit dem Primer-Paar 2048 [5' GAA TRY GAT NTW NTW GTW GYW GGW WSW GG] und 2054 [5' NAR NCC NCC YTT NGT NCC] ein 1463 bp Fragment amplifiziert und in pCRScript^{™} Amp SK(+) [Stratagene] kloniert. Mit dem Insert als DNA-Sonde wurden überlappende genomische Klone aus einer DNA-Bibliothek, die unter Verwendung des Zero Background^{™} / Kan Cloning Kits [Invitrogen] hergestellt worden war, isoliert. Die Sequenz des *Bacillus sphaericus* 3-Ketosteroid-Δ¹-Dehydrogenasegens wurde mit einem GATC^{®} 1500 Sequencer [GATC] bestimmt. Die von der Gensequenz abgeleitete Proteinsequenz ist mit der Sequenz der 3-Ketosteroid-Δ¹-Dehydrogenase aus *Comamonas testosteroni* zu 34 % identisch. Die Ähnlichkeit beträgt 54 %. Zur 3-Ketosteroid-Δ¹-Dehydrogenase aus *Arthrobacter simplex* besteht eine Identität von 34 % und eine Ähnlichkeit von 54 %.

### Beispiel 2

### Isolierung und Charakterisierung der Promotor- und Terminator-Sequenzen

Als regulatorische Sequenzen für die Überexpression der 3-Ketosteroid-Δ¹-Dehydrogenasegene wurden Promotor- und Terminatorelemente des 3-Ketosteroid-Δ¹-Dehydrogenasegens aus *Bacillus sphaericus* verwendet. Beide Elemente wurden im Zuge der Klonierung des Gens isoliert und charakterisiert.
Der Promotor enthält an Position 84 bp bzw. 61 bp oberhalb des Startcodons zwei Hexanucleotide [TTGACT-84 bis -79 / TATACT-_{61bis-56}], die mit jeweils einer Abweichung dem Consensus bakterieller Promotoren entsprechen [ -10 / -35 Box ]. Die Distanz von 17 Nucleotiden der beiden Elemente zueinander entspricht exakt dem bakteriellen Consensus [s. Record MT et al. (1996) In: *Escherichia coli and Salmonella,* Neidhardt FC (ed), 2^{nd} Edition, ASM Press, Washington DC, Vol 1, pp 792-821].
16 bp oberhalb des Startcodons liegt eine für *Bacillus* typische Ribosomen-Bindestelle [AGGGAGG_{-16 bis-10}; Band L and Henner DJ (1984) *DNA* **3**: 17-21]. Promotoraktivität wurde für Fragmente von Position -126 [*Sal*I] bis Position -28 [*Cla*I] und von Position -258 *[Pst*l] bis Position -28 [Clal] in *lac*Z-Assays nachgewiesen.

9 bp hinter dem Stopcodon liegt ein Palindrom [AAGCCCTTCCT₁₆₉₈₋₁₇₀₈/AGGAAGGGCT₁₇₃₁₋₁₇₄₁], das als p-unabhängiger Terminator fungiert [s. Richardson JP and Greenblatt J (1996) In: *Escherichia coli and Salmonella,* Neidhardt FC (ed), 2^{nd} Edition, ASM Press, Washington DC, Vol1, pp 822-848].
Prinzipiell können auch andere Promotoren und Terminatoren verwendet werden [s. u.a. Doi RH (1984) In: Biotechnology and Genetic Engineering Reviews, Vol 2, Russell GE (ed), Intercept, Newcastle Upon Tyne, UK, pp 121-153; Le Grice SFJ et al. (1986) In: *Bacillus Molecular Genetics and Biotechnology Applications,* Ganesan AT and Hoch JA (eds), Academic Press, New York, 433-445; Mountain A (1989) In: *Bacillus,* Harwood CR (ed), Plenum Press, New York, pp 73-114; Le Grice SFJ (1990) *Meth Enzymol* 185:210-214; Wang and Doi (1992) In: *Biology of Bacilli: Applications to Industry,* Doi et al. (eds), Massachusetts, Butterworth-Heinemann, pp 143-188].

### Beispiel 3

### Konstruktion der Expressionsplasmide

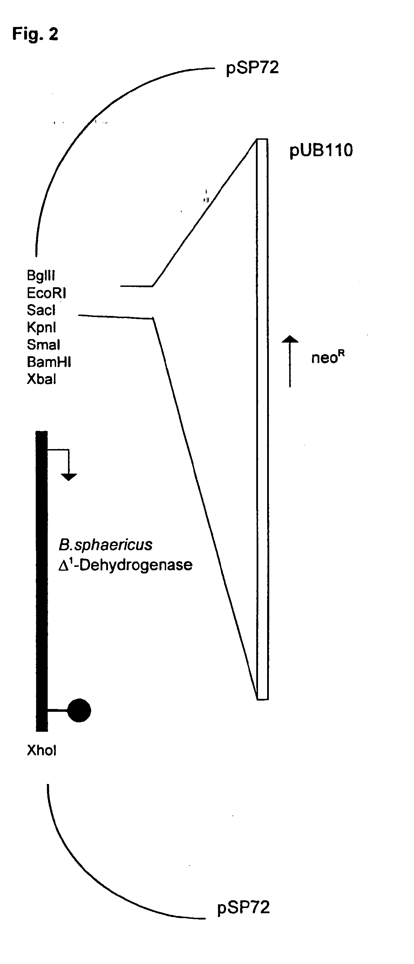

Für die Herstellung eines Expressionsplasmids wurde zunächst ein "shuttle"-Plasmid, bestehend aus pSP72 [Promega] und Teilen aus pUB110 [McKenzie et al. (1986) *Plasmid* **15**:93-103], konstruiert. Hierzu wurde pUB110 mit *Eco*RI und *Pvu*lI restringiert und das entstehende 3,6 kb Fragment in die *Eco*RI und *Eco*RV Schnittstellen von pSP72 inseriert. Das 3-Ketosteroid-Δ¹-Dehydrogenasegen von *Bacillus sphaericus,* flankiert von Promotor- und Terminationssequenzen [Position - 126 (Sa/l) bis Position 1861 (Scal)], wurde als Xbal-Scal-Fragment in die *Xbal* und Pvull Schnittstellen des oben beschriebenen "shuttle"-Vektors ligiert [→ TS#196, s. Abb. 2].
Ein zweites Expressionsplasmid trägt einen modifizierten Δ¹-Dehydrogenasegen-Promotor p(Δ¹)ₘᵤₜ: Durch PCR-Mutagenese wurden in der -35 [TTGACT → TTGACA] und in der -10 Box [TATACT → TATAAT] jeweils eine Base ausgetauscht, um eine exakte Übereinstimmung mit dem Consensus bakterieller Promotoren zu erzielen. Hierfür wurde der Promotor zunächst mit dem Mutagenese-Primer 2089ₘᵤₜ [CCA TCG ATG AAT CTG GTC TTC CTA TTA AAA ATT ATA GAA TTA AAC TAA TAT TCT GTC AAT TTT TCC_{-29bis-91}] und Primer 2090 [CAT GAC AAA ATT ATT TGA TTT AAT CAC_{-258 bis-284}] amplifiziert und als *Pst*l-*Cl*a*l*-Fragment in die entsprechenden Schnittstellen von pBluescript II KS(+) inseriert. Die Mutationen wurden durch Sequenzanalyse verifiziert. p(Δ¹)ₘᵤₜ wurde als *Xb*aI-ClaI-Fragment ausgeschnitten und in die entsprechenden Schnittstellen von TS#196 ligiert. Hierbei wurde der *wt-*Promotor gegen p(Δ¹)ₘᵤₜ ausgetauscht [→ TS#251].
Zwei weitere Plasmide tragen zusätzlich ein Plasmid stabilisierendes Signal, *parS* [Lin DC and Grossman AD (1998) *Cell* **92**:675-685]. Dieses wurde über zwei zueinander komplementäre Oligonucleotide, 2091*_{parS}* [GAT CCT GTT CCA CGT GAA ACA G] und 2092*_{parS}* [GAT CCT GTT TCA CGT GGA ACA G], in die *Bam*HI Schnittstelle von TS#196 [→ AD#82] und TS#251 [→ TS#255] kloniert.
Zur Expression in *Escherichia coli* DH5α [≡ DSM 6897] wurde das 3-Ketosteroid-Δ¹-Dehydrogenasegen von *Bacillus sphaericus,* flankiert von Promotor- und Terminationssequenzen als 2865 bp Sall-partieIISau3A-Fragment [Position -126 bis Position 2739] in das mit BamH1 und Xhol geschnittene Plasmid pZErO^{™}-2 kloniert und in *Escherichia coli* DH5α transformiert [→ Plasmid MS#46 bzw. Stamm MS#46_{MS#46}].

### Beispiel 4

### Erzeugung rekombinanter Stämme der Gattung Bacillus für die Einführung einer Δ¹-Dehydrierung am Steroid

Die Expressionsplasmide TS#196, TS#251, AD#82 und TS#255 wurden in *Bacillus subtilis* DSM 402 [Deutsche Stammsammlung für Mikroorganismen, Braunschweig] und *Bacillus sphaericus* ATCC 13805 transformiert. *Bacillus subtilis* und *Bacillus sphaericus* sind *gram-positive,* apathogene Organismen. Sie sind einfach zu kultivieren. Im Gegensatz zu *Bacillus sphaericus* ist *Bacillus subtilis* molekulargenetisch gut charakterisiert. Es gibt eine Reihe von Beispielen für die heterologe Expression und Sekretion von Proteinen zur Herstellung rekombinanter Genprodukte [Wang and Doi (1992) In: *Biology of Bacilli: Applications to Industry,* Doi et al. (eds), Massachusetts, Butterworth-Heinemann, pp 143-188]. Auch werden hier geeignete Promotoren und Terminatoren beschrieben.
Mit einigen der rekombinanten Stämmen wurden exemplarisch Umsetzungen eines Gemischs aus Hydrocortison [F], Hydrocortison-17-acetat [MAF] und Hydrocortison-21-acetat [EAF] zu Prednisolon [Pln] im Schüttelkolben durchgeführt. Neben den Ausgangssubstanzen F, MAF und EAF sowie dem gewünschten Produkt Pln wurde zusätzlich die Bildung von Prednisolon-21-acetat [Pln-21-Acetat] und der unerwünschten Nebenzone 11β-Hydroxyandrosta-1,4-dien-3,17-dion [11β -OH-ADD] verfolgt. Um das Umsetzungspotential der rekombinanten Stämme zu demonstrieren, wurde mit Substratkonzentrationen gearbeitet, bei denen *Bacillus sphaericus* ATCC 13085 nicht mehr als 20 % Pin bildet.
Die Stämme AD#67_{TS#196'} AD#94_{TS#251}, AD#95_{TS#255}, AD#96_{TS#255}, AO#116_{TS#251} und AO#205_{TS#196} sind aus *Bacillus sphaericus* ATCC 13085 hervorgegangen und enthalten jeweils das angezeigte Expressionsplasmid. Die Stämme AD#89_{TS#196} und AD#90_{TS#196} sind aus *Bacillus subtilis* DSM 402 hervorgegangen und enthalten jeweils das angezeigt Expressionsplasmid.

Die nachfolgenden Umsetzungsbeispiele beschreiben die mikrobiologische Ausführbarkeit der Erfindung, ohne diese auf die Beispiele zu beschränken.

### Beispiel 1

### Umsetzung von EAF/MAF/F zu Pln

*Bacillus sphaericus* ATCC 13805, AD#67_{TS#196}, AD#94_{TS#251}, AD#95_{TS#255}, AD#96_{TS#255}, AD#116_{TS#251}, *Bacillus subtilis* DSM 402, AD#89_{TS#196,} AD#90_{TS#196,} *Escherichia coli* DH5α DSM 6897 und MS#46_{MS#46} wurden in LB-Medium [Sambrook et al. (1989) *Molecular Cloning: a laboratory manual,* Cold Spring Harbor Laboratory Press, Cold Spring Harbor, New York] in Anwesenheit von 5 µg/ml Neomycin [*Bacillus sphaericus* Derivate], 50 µg/ml bzw. 100 µg/ml Kanamycin [*Escherichia coli* bzw. *Bacillus subtilis* Derivate] oder ohne Zusatz von Antibiotikum [*wt*-Stämme] bei 37 °C und 220 rpm kultiviert. Zur Umsetzung von EAF/MAF/F zu Pln wurde das Impfmaterial 1:10 in frisches LB-Medium ohne Zusatz von Antibiotikum überführt und die Kultur wie oben geschüttelt. Prinzipiell kann auch jedes andere Medium verwendet werden, in dem der Organismus wachsen kann. Substratzugabe erfolgte nach 3 Stunden. Nach 24 Stunden wurden die Kolben abgenommen, Edukte und Produkt(e) extrahiert und HPLC-analysiert [s. Tab. 1; Reaktionschema s.u.]. *Bacillus subtilis* DSM 402 und *Escherichia coli* DH5α zeigen erwartungsgemäß keine Umsetzung, *Bacillus sphaericus* ATCC 13085 bildet nach 24 Stunden weniger als 20 % Produkt, während alle rekombinanten Stämme der Gattung *Bacillus* [AD#67_{TS#196}, AD#94_{TS#251}, AD#95_{TS#255}, AD#96_{TS#255}, AD#89_{TS#196} und AD#90_{TS#196}] im gleichen Zeitraum mehr als 80 % Pln hervorbringen. Ein Abbau von Substrat oder Produkt über 48 Stunden konnte nicht beobachtet werden.
Alle im folgenden beschriebenen Versuche wurden exemplarisch mit AD#67_{TS#196} oder AD#116_{TS#251}, durchgeführt. Als Standard wurde *Bacillus sphaericus* ATCC 13085 eingesetzt. Die Versuche zeigen die um ein Vielfaches gesteigerte Umsetzungsaktivität o.g. rekombinanter Stämme hinsichtlich Δ¹-Dehydrierungen am Steroidmolekül.

### Beispiel 2

### Kinetik der Umsetzung von EAF/MAF/F zu Pln [1g/L]

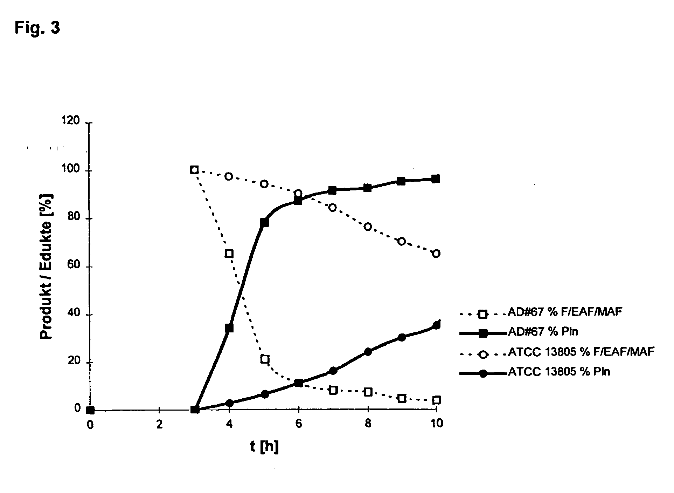

Zunächst wurde eine Δ¹-Dehydrierung am Beispiel einer Umsetzung von EAF/MAF/F zu Pln analog oben bei einer Substratkonzentration von 1 g/L im Schüttelkolben durchgeführt [LB-Medium, 37 °C, 220 rpm]. Die Substratzugabe erfolgte nach 3 Stunden. Um den Ablauf der Reaktion verfolgen zu können, wurden nach 4, 5, 6, 7, 8, 9, 10, 11, 12 und 24 Stunden Proben genommen, Edukte und Produkt extrahiert und HPLC-analysiert. Während der Stamm ATCC 13805 24 Stunden benötigt, um das Substrat vollständig in Pln umzuwandeln, hat Stamm AD#67 die entsprechende Menge an Pln bereits nach <10 Stunden gebildet [Abb. 3; Reaktionschema s.u.].

### Beispiel 3

### Kinetik der Umsetzung von EAF/MAF/F zu Pln [10 g/L]

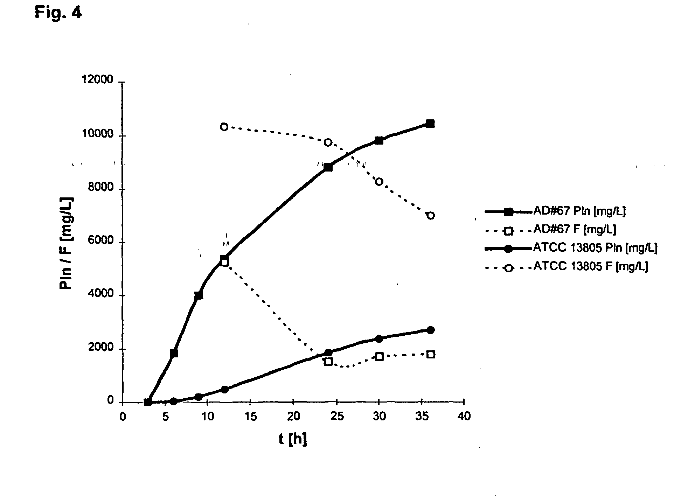

Der gleiche Versuch wurde bei einer Substratbelastung von 10 g/L durchgeführt. Die . Substratzugabe erfolgte nach 3 Stunden, Proben wurden nach 6, 9, 12, 24, 30 und 36 Stunden genommen, die Steroide extrahiert und analysiert. Nach 6 Stunden weist die ATCC 13805 Kultur nur 1 % Pln auf, während der Stamm AD#67 bereits > 15 % Produkt gebildet hat. Nach 12 Stunden hat Stamm AD#67 bereits mehr als 50 % des Substrats in Pln umgewandelt, Stamm ATCC 13805 dagegen nur 5 % [Abb. 4; Reaktionschema s.u.].

Die hohe Umsetzungsaktivität von Stamm AD#67 beschränkt sich nicht auf das Verfahren zur Herstellung von Prednisolon aus EAF/MAF/F, sondern gilt generell für die Einführung von Δ¹ in Steroid-Moleküle.

### Beispiel 4

### Umwandlung von 4-Androsten-3,17-dion [AD] zu Androsta-1,2-dien-3,17-dion [ADD]

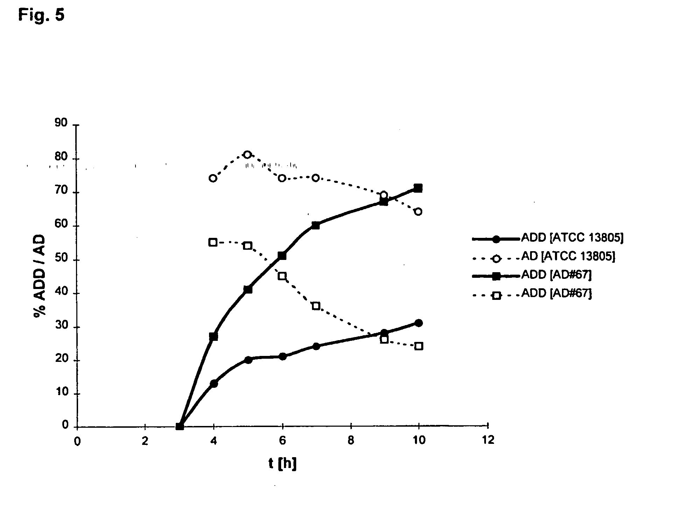

Die Umwandlung von AD zu ADD durch Stamm AD#67 bzw. Stamm ATCC 13805 wurde analog oben im Schüttelkolben untersucht [LB-Medium, 37 °C, 220 rpm]. Die Substratzugabe erfolgte nach 3 Stunden, Proben wurden nach 4, 5, 6, 7, 9 und 10 Stunden genommen. Wie bei der Umwandlung von MAF/F zu Pln erfolgt die Produktbildung bei Fermentation mit Stamm AD#67 erheblich schneller als bei Verwendung von Stamm ATCC 13805. *Bacillus sphaericus* ATCC 13805 hat nach 10 Stunden weniger als 30 % des Substrats zu ADD umgewandelt, während bei Stamm AD#67 zu diesem Zeitpunkt bereits mehr als 70 % Produkt isoliert werden konnten [Abb. 5].

### Beispiel 5

### Umsetzung von Fluocortolon A Acetat [FCAA] zu Fluocortolon [FC]

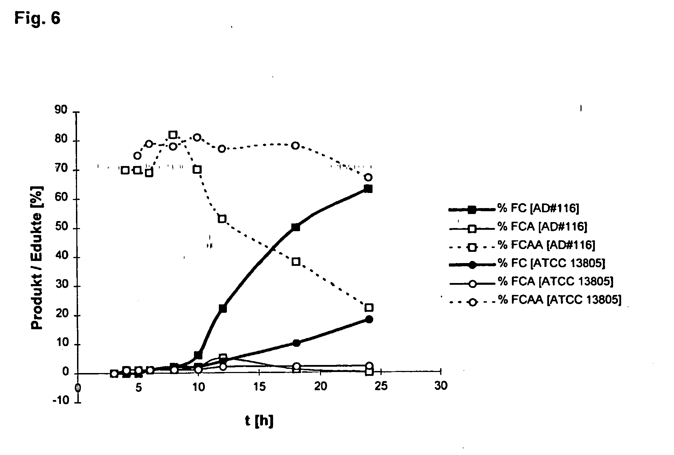

Auch fluorierte Steroide werden durch rekombinante Stämme wesentlich effizienter an Position 1 dehydriert als es bislang mit den zur Verfügung stehenden Biokatalysatoren möglich gewesen ist Dies zeigt die Umwandlung von FCAA zu FC analog oben im Schüttelkolben durch AD#116 im Vergleich zu *Bacillus sphaericus* ATCC 13805 [Abb. 6; Reaktionschema s.u.].

### Beispiel 6

### Umsetzung von 11β,17α-Dihydroxy-6α,9α-difluor-16α-methylprogesteron [DDFMP] zu Δ¹-11β,17α-Dihydroxy-6α,9α-difluor-16α-methylprogesteron [Δ¹DDFMP]

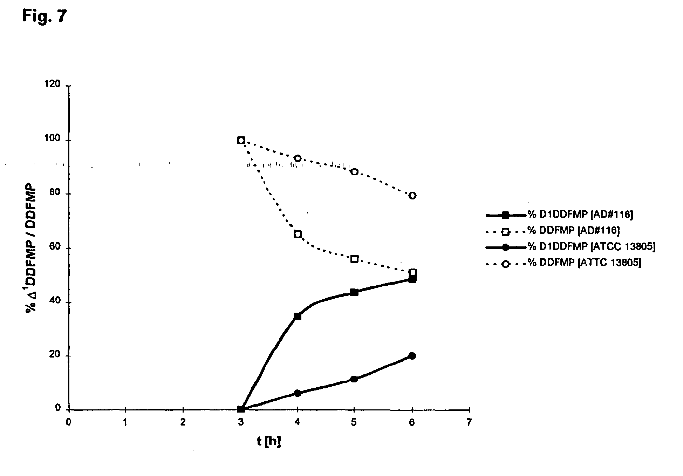

Auch die Umwandlung von DDFMP zu Δ¹DDFMP analog obiger Beispiele erfolgt erheblich effizienter mit AD#116 als mit *Bacillus sphaericus* ATCC 13805 [Abb. 7; Reaktionsschema s.u.].

### Beispiel 7

### Umwandlung von EAF/MAF/F zu Pln im 10 L Fermenter [20 g/L] Vergleich Stamm AD#67 / Bacillus sphaericus ATCC 13805

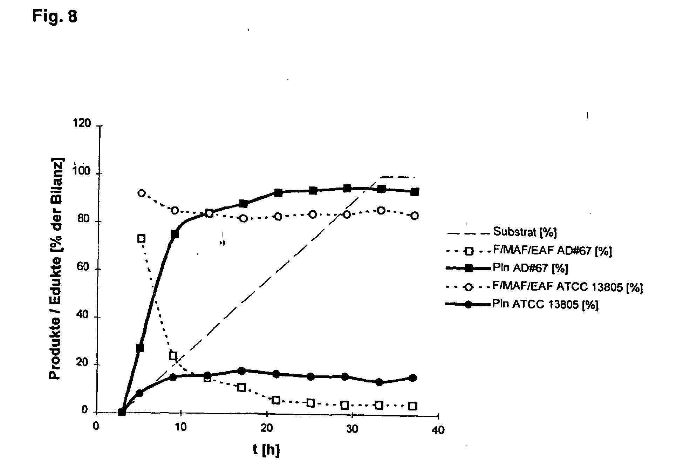

Die Δ¹-Dehydrierungskapazität von Stamm AD#67 im Vergleich zu *Bacillus sphaericus* ATCC 13805 wurde am Beispiel EAF/MAF/F → Pln im 10 L Fermenter getestet. Die Reaktion wurde bei einer 20-fach höheren Substratbelastung durchgeführt. Die Anzucht des Impfmaterials erfolgte in einem ersten Schritt über Nacht bei 37 °C und 220 rpm in LB-Medium in Anwesenheit von 5 µg/ml Neomycin [AD#67] bzw. ohne Zusatz eines Antibiotikums [ATCC 13805]. Im Anschluß daran wurde die Übernachtkultur 1:100 in eine 1000 ml Zwischenkultur überführt und für 9 h bei 37 °C und 220 rpm bis zu einer optischen Dichte von 2,4 geschüttelt. Die Fermentation erfolgte in LB-Medium ohne Zusatz eines Antibiotikums. Prinzipiell kann jedoch jedes andere Medium verwendet werden, in dem der Organismus wachsen kann. Das Substrat wurde nach 3 Stunden kontinuierlich über 30 Stunden hinzugegeben. Der pH-Wert wurde bei pH 8 gehalten. Im Verlauf der Fermentation wurden Proben gezogen und auf ihren Gehalt an Produkt und Edukt getestet. Das Fermentationsprofil zeigt, daß *Bacillus sphaericus* ATCC 13805 Substratkonzehtrationen dieser Größenordnung nicht bewältigen kann: Die Reaktion stoppt bei einem Rest an Substrat von über 80 %. Die Umwandlungskapazität von Stamm AD#67 ist dagegen beträchtlich: Kurz nach Abschluß der Substratapplikationsphase ist die Reaktion nahezu vollständig [>98 %] abgelaufen [Abb. 8]. Die Umwandlungsaktivität von Stamm AD#67 liegt bei 0,6 g/L pro Stunde. Stamm ATCC 13805 zeigt dagegen eine Aktivität von 0,1 g/L pro Stunde. Störende Nebenzonen wie z.B. 11-β-OH-ADD wurden allenfalls in Spuren beobachtet. Die Kristallisatausbeute an Pln lag bei über 80 % der Theorie und entspricht dem Wert, der beim herkömmlichen Verfahren erzielt wird [Reaktionschema s.u.].

### Beispiel 8

### Umsetzung von 11β,21-Dihydroxy-2'-methyl-5'βH-pregn-4-eno[17,16-d]oxazol-3,20-dion zu 11β,21-Dihydroxy-2'-methyl-5'βH-pregna-1,4-dieno[17,16-d]oxazol-3,20-dion (Deflazacortalkohol)

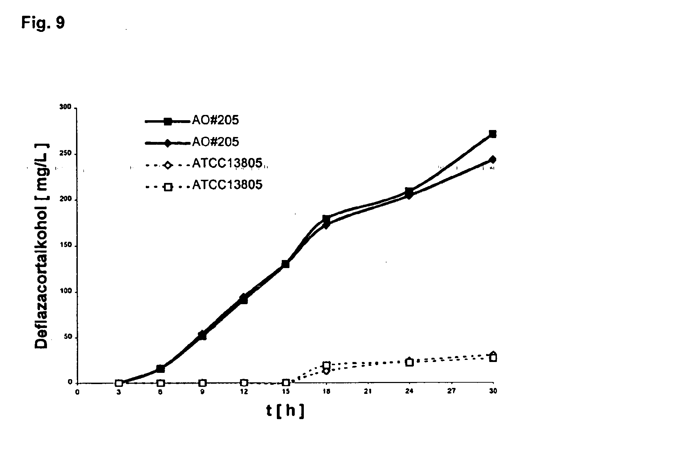

Auch die Umwandlung von 1 g/L 11β,21-Dihydroxy-2'-methyl-5'βH-pregn-4-eno[17,16-d]oxazol-3,20-dion zu Deflazacortalkohol im Schüttelkolben analog obiger Beispiele erfolgt erheblich effizienter mit AO#205 als mit *Bacillus sphaericus* ATCC 13805 [Abb. 9; Reraktionsschema s.u.]. Abweichend zu oben wurde eine Medium bestehend aus 12 g/L 67 %-igem Hefextrakt, 27 g/L Maisquellwasser und 9.2 g/L NaCl verwendet.

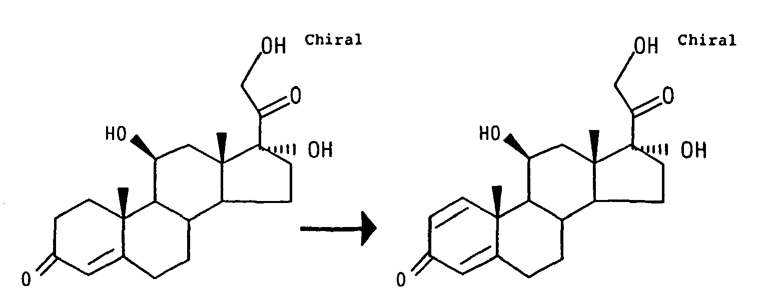

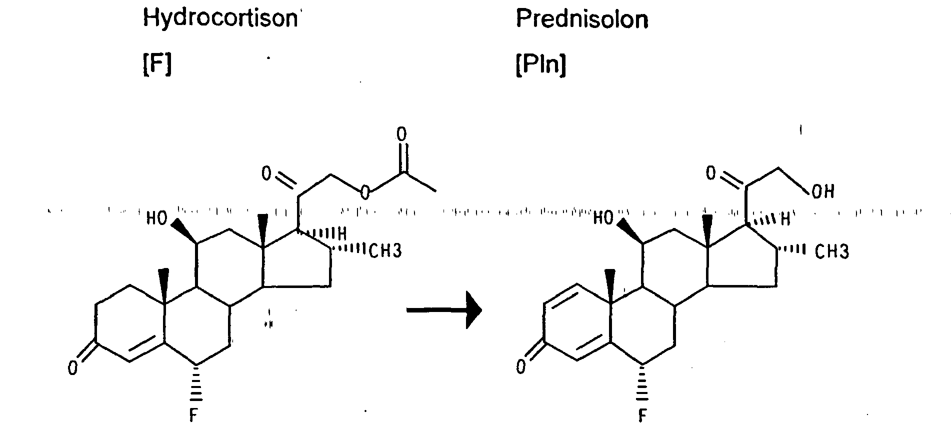

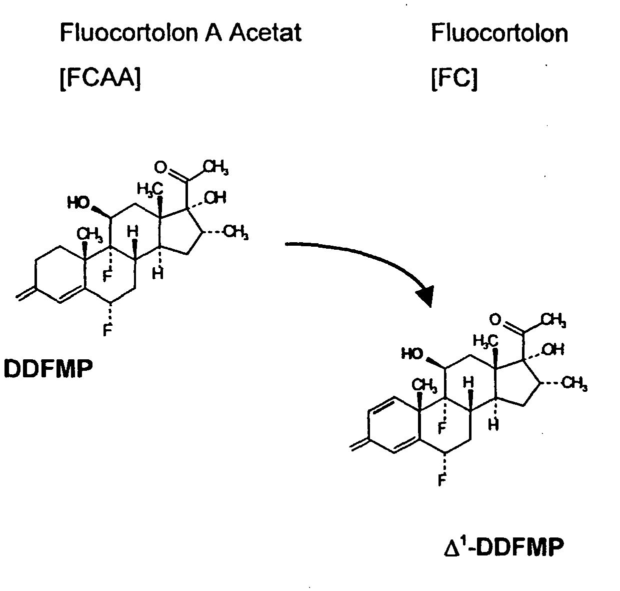

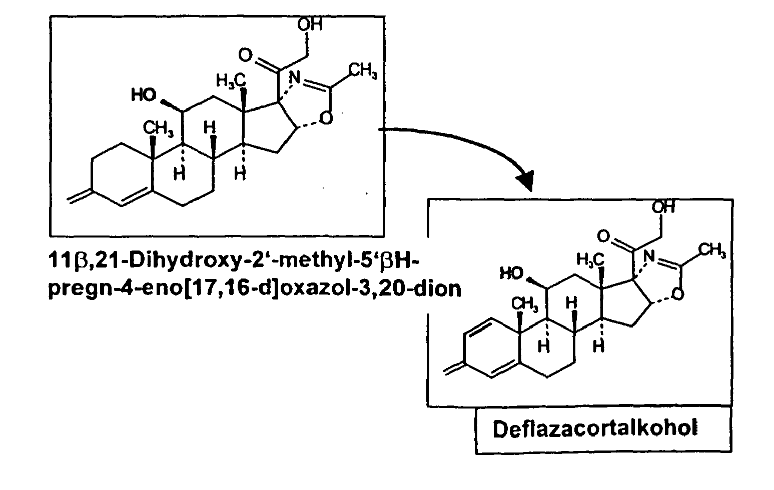

| **Tabelle 1** | | | | | |
|---|---|---|---|---|---|
| **Stamm** | **F [mg/L]** | **EAF/MAF [mg/L]** | **Pin [mg/L]** | **11β-OH-ADD [mg/L]** | **Substratbelastung** |
| *Bacillus sphaericus* | | | | | |
| ATCC 13805^{a)} | 7720 | 39 | 1730 | <10 | 9 g/L |
| AD#67_{TS#196}^{a)} | 1570 | 22 | 7790 | <10 | 9 g/L |
| AD#94_{TS#251}^{a)} | 1650 | 30 | 7480 | 13 | 9 g/L |
| AD#95_{TS#255}^{a)} | 1460 | 18 | 7500 | 13 | 9 g/L |
| AD#96_{TS#255}^{a)} | 1530 | 19 | 7130 | 13 | 9 g/L |
| AD#116_{TS#251} | 2150 | n.b.^{b)} | 9330 | <10 | 12 g/L |
| | | | | | |
| *Bacillus subtilis* | | | | | |
| DSM 402^{a)} | 9030 | 510 | <1 | <10 | 9 g/L |
| AD#89_{TS#196} | 1820 | 500 | 8280 | 14 | 10 g/L |
| AD#90_{TS#196} | 1680 | 580 | 8120 | <10 | 10 g/L |
| | | | | | |
| *Escherichia coli* | | | | | |
| DH5α | 11110 | 1020 | <1 | n.b.^{b)} | 12 g/L |
| MS#46_{MS#46} | 9510 | 1080 | 1910 | n.b.^{b)} | 12 g/L |

| | | | | | |
|---|---|---|---|---|---|
| ^{a)} Doppelbestimmung ^{b)} nicht bestimmt | | | | | |

### SEQUENCE LISTING

(1) GENERAL INFORMATION:
   (i) APPLICANT:
      (A) NAME: Schering Aktiengesellschaft
      (B) STREET: Müllerstrasse 178
      (C) CITY: Berlin
      (E) COUNTRY: Germany
      (F) POSTAL CODE (ZIP): D-13342
      (G) TELEPHONE: (030)-9681-2085
      (H) TELEFAX: (030)-4681-2058
   (ii) TITLE OF INVENTION:
      Verfahren zur Überexpression von Dehydrogenasen
   (iii) NUMBER OF SEQUENCES: 20
   (iv) COMPUTER READABLE FORM:
      (A) MEDIUM TYPE: Floppy disk
      (B). COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER:
   (vi) PRIOR APPLICATION DATA:
      (A) APPLICATION NUMBER:
      (B) FILING DATE:
(2) INFORMATION FOR SEQ ID NO: 1:
   (i) SEQUENCE CHARACTERISTICS:
      (A) LENGTH: 1506 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (A) ORGANISM: Arthrobacter *simplex* ATCC 6946
      (C) INDIVIDUAL ISOLATE: 3-Ketosteroid-Δ¹-Dehydrogenase-Gen *ksdD*
   (vii) IMMEDIATE SOURCE:
      (A) LIBRARY: EMBL Datenbank D37969
         (Molnar I et al., 1995, Mol Microbiol 15:895-905)
   (viii) POSITION IN GENOME:
      (B) MAP POSITION: from 1435 to 2982 coding region
      (C) UNITS:
   (xi) SEQUENCE DESCRIPTION: SEQ ID NO. 1:
(2) INFORMATION FOR SEQ ID NO: 2:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE
      (A) ORGANISM:
      (C) INDIVIDUAL ISOLATE: Primer 2026
   (vii) IMMEDIATE SOURCE:
      (A) LIBRARY: EMBL/ GenBank, AC:D37969
   (viii) POSITION IN GENOME:
      (B) MAP POSITION: from 8 to 32 coding region (Primer)
      (C) UNITS:
   (xi) SEQUENCE DESCRIPTION: SEQ ID NO. 2:
      GAT CAC GAT GGA CTG GGC AGA GGA GTA CGA CG ₁₄₂₈₋₁₄₅₉
(2) INFORMATION FOR SEQ ID NO: 3:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (A) ORGANISM:
      (C) INDIVIDUAL ISOLATE: Primer 2017
   (vii) IMMEDIATE SOURCE:
      (A) LIBRARY: EMBL/ GenBank, AC:D37969
   (viii) POSITION IN GENOME:
      (B) MAP POSITION: from 1 to 34 coding region (Primer)
      (C) UNITS:
   (xi) SEQUENCE DESCRIPTION: SEQ ID NO. 3:
      GAC GCC GTA CTT CTG GCG GAG CTC GTC ATT GGC C₂₁₇₅₋₂₁₄₂
(2) INFORMATION FOR SEQ ID NO: 4:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (A) ORGANISM:
      (C) INDIVIDUAL ISOLATE: Primer 2026
   (vii) IMMEDIATE SOURCE:
      (A) LIBRARY: EMBL/ GenBank, AC:D37969
   (viii) POSITION IN GENOME:
      (B) MAP POSITION: from 9 to 42 coding region (Primer)
      (C) UNITS:
   (xi) SEQUENCE DESCRIPTION: SEQ ID NO. 4:
      CGG GAT CCA TGG ACT GGG CAG AGG AGT ACG ACG TAC TGG TGG ₁₄₃₅₋₁₄₆₈ BamHI NcoI
(2) INFORMATION FOR SEQ ID NO: 5:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (A) ORGANISM:
      (C) INDIVIDUAL ISOLATE: Primer
   (vii) IMMEDIATE SOURCE:
      (A) LIBRARY: EMBL/ GenBank, AC:D37969
   (viii) POSITION IN GENOME:
      (B) MAP POSITION: from 9 to 42 coding region (Primer)
      (C) UNITS:
   (xi) SEQUENCE DESCRIPTION: SEQ ID NO. 5:
      CGG AAT TCT CAT CGC GCG TCC TCG GTG CCC ATG TGC CGC ACG₂₉₈₂-₂₉₄₉ EcoRI
(2) INFORMATION FOR SEQ ID NO: 6:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (A) ORGANISM:
      (C) INDIVIDUAL ISOLATE: Primer 2032
   (vii) IMMEDIATE SOURCE:
      (A) LIBRARY: EMBL/ GenBank, AC:D37969
   (viii) POSITION IN GENOME:
      (B) MAP POSITION: from 1 to 19 coding region (Primer)
      (C) UNITS:
   (xi) SEQUENCE DESCRIPTION: SEQ ID NO. 6:
      CGA TCG TCG AGA CCG ACG G ₂₀₆₆₋₂₀₈₄
(2) INFORMATION FOR SEQ ID NO: 7:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (A) ORGANISM:
      (C) INDIVIDUAL ISOLATE: Primer 2055
   (vii) IMMEDIATE SOURCE:
      (A) LIBRARY: EMBL/ GenBank, AC:D37969
   (viii) POSITION IN GENOME:
      (B) MAP POSITION: from 1 to 27 coding region (Primer)
      (C) UNITS:
   (xi) SEQUENCE DESCRIPTION: SEQ ID NO. 7:
      GCA GCA CCG GGT TCG CGG GGA ACC AGG ₁₆₁₈₋₁₅₉₂
(2) INFORMATION FOR SEQ ID NO: 8:
   (i) SEQUENCE CHARACTERISTICS:
      (A) LENGTH: 3630 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (A) ORGANISM: *Arthrobacter simplex* ATCC 6946
      (C) INDIVIDUAL ISOLATE: 3-Ketosteroid-Δ¹-Dehydrogenase-Gen *ksdD*
         3-Ketosteroid-Δ⁵-Isomerase-Gen *ksdI*
   (vii) IMMEDIATE SOURCE:
      (A) LIBRARY: EMBL/ GenBank, AC:D37969
   (viii) POSITION IN GENOME:
      (B) MAP POSITION: from 1435 to 2982 ksdD coding region
         from 2979 to 3350 ksdI coding region
      (C) UNITS:
   (xi) SEQUENCE DESCRIPTION: SEQ ID NO. 8:
   ctgcaggagc tcggcctggt cgagcgggcc gcggacacct tcgaccggcg caccacgctg    60
   gtccgctgct cgcgccgcgg cgtcgcccag gtacgccggc tcgcggccgc ccagcgcgcc   120
   gacctagccg ccgcgctcgg tccggtcgac ccggccgacc gggaccgctg gacggtgctc   180
   gtggagcgct acgtgcgggc tctcgaggcc cgcgggctca tctccgagct gtgactcgcc   240
   ggtaagttca gagaacatta tgtgcaaacg gtccagtaaa actagccgtt cggcaagtag   300
   attggtgacc catcgcattc tgtgtttccg caggtcagag gcacagtttc ggaggtgacc   360
   gcagtcccgg tgaccgggag tgccgattca cggcggaaac ctcaccgaaa aatatgtgcg   420
   ttcgatccac ttgatttgcc ctgtgtcagt gctcacactc gacgggaggc cgcactcccg   480
   aggagcaccc gcatgaccgt caccgcactg cccacgacca cgcccgccgg ctccggcgca   540
   cccgccctgg accccgacga ccgccgcacg cccctgggcg tcgtgggccg ggtgacccgg   600
   atcctcaacg ccttcagcga gtcccccgac cgcctcatgc tcgaggacgt gatggcgctg 660
   accggcctgc cccggtcgac cgccttccgg atcctcggcc agctcatcga cgaggggtgg 720
   gtcgagcacg acacccgcgg ctaccggctc gggccgcacg cgcccacgct caccggccgg 780
   cccggcgagc accaggaggt gcgggtcgcc gcgtcgccgt acctcaacga gctgcacgcc 840
   ctcaccggcg cggtcgccca cctctcggtg ctcgagggcg accgggtcca ctacctcgac 900
   aagatcggcg gctccgcggc tcgcgccgtc ccctcgcggg tcggcgcccg gctgctcgcc 960
   tccgacaccg tcagcggccg cgcgctgctc gcctgccgct cccccgagta cgtcgacgac 1020
   gtcctcggcc cgcggctgcc cgcgccccgg ctcgccctgc tccaccgcga cctcgccgcc 1080
   gcccgccagc gccgcggcgt cgtgcacgcc ccggccgacc cgaccaccgg catcgcctcg 1140
   atcgccgcac ccgtcctcgg cccgcacgga gccgtcgccg cgatctcgct ggccctgccc 1200
   ggcgagctgc cgcccgcccg gctcgcaccc ctgctgctca accaggccca ccggatcgcc 1260
   ggcgtcctgt tcccccagcg ccgcctgcac ggacgatcct ggctgcgctg atcccgcccc 1320
   cgcccggaga ctcccgcagg acgggagaac ccaccggggc acccggggcc gctgcctagc 1380
   gtcgccgcca cgacgccgga ggtcggcgtc ggtcaacccg gcgagaggat cacgatggac 1440
   tgggcagagg agtacgacgt actggtggcg ggctccggcg ccggcggcat ggccgggacc 1500
   tacaccgcgg cccgcgaggg gctcagcgtg tgcctggtcg aggccgggga caagttcggc 1560
   gggacgaccg cctactccgg cggcggtggg gcctggttcc ccgcgaaccc ggtgctgctg 1620
   cgggcgggca ccgacgacac gatcgaggac gctctcgagt actaccgagc ggtcgtcggc 1680
   gaccgcaccc ccgcggacct gcaggagacc tacgtccgcg gcggcgccgg cctggtcgcc 1740
   tacctcgagg aggacgacca cttctccttc gagtcctacc cgtggccgga ctacttcggc 1800
   gacgccccca aggcccgtcg cgacggccag cggcacatca tcccgacgcc gctgccggtg 1860
   ccctccgcac ccgagctgcg cgaggtggtc cgcgggccgc tcgacaacga ccggctcggc 1920
   acgccgcagc ccgacgacct gttcatcggc ggacgggcgc tcgtcgcccg cttcctgacc 1980
   gcgctcgcga cctaccccca cgccacgctc gtgcgcgaga ccgcactggc cgagctcgtc 2040
   gtcgaggacg gcgtcgtggt cggcgcgatc gtcgagaccg acggcgtccg ccgcgcgatc 2100
   cgggcccgcc gcggcgtcct cctggccgcg ggcggcttcg aggccaatga cgagctccgc 2160
   cagaagtacg gcgtccccgg cgtcgcgcgc gacacgatgg gcccgccgac caacgtcggc 2220
   gccgcgcacc aggccgcgat cgcggtcggc gccgacaccg acctgatggg cgaggcctgg 2280
   tggtcccccg ggctgaccca ccccgacgga cgatcggcgt tcgcgctctg gttcaccggc 2340
   ggcatcttcg tcgacggcgc cggccggcgc ttcgtcaacg agtcggcgcc gtacgaccgg 2400
   ctcggccgcg ccgtcatcga ccacctcacc gagggcggcg tcaccccgcg gtactggatg 2460
   gtctacgacc acaaggaggg ctcgatcccc ccggtgcgcg ccaccaacgt ctcgatggtc 2520
   gacgaggagc agtacgtcgc cgcgggcctg tggcacaccg ccgacacgct gcccgagctg 2580
   gccgcgctga tcggcgtccc cgccgacgcg ctggtcgcca cggtcgcgcg cttcaacgag 2640
   ctcgtcgccg acgggtacga cgcggacttc ggccgcggcg gcgaggccta cgaccggttc 2700
   ttctccggcg gcgagccgcc gctggtgagc atcgacgagg ggccgttcca cgcggccgcc 2760
   ttcggcatct ccgacctcgg caccaagggc gggctgcgca ccgacacgtc cgcgcgcgtg 2820
   ctgaccgcgg acggcacgcc gatcgggggc ctctacgcag ccggcaatac gatggcggcg 2880
   ccgagcggca ccacctaccc gggcggtggc aacccgatcg ggacaagcat gctcttcagc 2940
   cacctcgccg tgcggcacat gggcaccgag gacgcgcgat gagcgccgag gtgaaggccg 3000
   ccgtggcgcg ctacctcgat gctgtcgccg gcggctcgcc ggccgcgatc gccgcgctct 3060
   acgcccccga cgccacgctc gaggaccccg tcggcgccga cctcgtccgc ggccgcgcgg 3120
   cgatcgaaga gttctacggc gccctcgccg gcgcgaaggt cagcaccgag ctgctcgccg 3180
   tccgcgccgt cgcgggccac gccgcgttct cgttccgggt caccaccgac gccggcgacc 3240
   agcagtacgt cgtcgagccg atcgacgtga tgacgttcga cgcggacggc cagatcacgt  3300
   ccatgcgggc gttctgggcg cccggggaca tggtcgtcac gccggcctga cggtcccgct  3360
   gtaacacgct gtccaccgcg cttcccggcg gttgtcgacg cgctctcggc gtgtcgcacg  3420
   gcgtgtcgcg ccgtggacag cgtgttacag cggcgggggc cgtcaggcgg tggccgcgtg  3480
   ggtggcgacg atgtggccga agaccagacc ctggccgatg gtcgcgccgg cccccgggta  3540
   gctgcgcccg aagacgttgc ccgcggtgtt gccgatcgcg tagagcccct cgatcgggct  3600
   gccgtcggcg cgcagcggac ggccgagctc                                  3630
(2) INFORMATION FOR SEQ ID NO: 9:
   (i) SEQUENCE CHARACTERISTICS:
      (A) LENGTH: 241 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-.SENSE: No
   (vi) ORIGINAL SOURCE:
      (A) ORGANISM: *Bacillus sphaericus* ATCC 13805
      (C) INDIVIDUAL ISOLATE: promoter of 3-Ketosteroid-Δ¹-Dehydrogenase *ksdD*
         (*ksd* ⇒ keto-steroid-degradation)
   (vii) IMMEDIATE SOURCE:
      (A) LIBRARY: --
   (viii) POSITION IN GENOME:
      (B) MAP POSITION: from -258 to -28 promoter region
      (C) UNITS:
   (xi) SEQUENCE DESCRIPTION: SEQ ID NO. 9:
(2) INFORMATION FOR SEQ ID NO: 10:
   (i) SEQUENCE CHARACTERISTICS:
      (A) LENGTH: 1884 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (A) ORGANISM: *Bacillus sphaericus* ATCC 13805
      (C) INDIVIDUAL ISOLATE: 3-Ketosteroid-Δ¹-Dehydrogenase *ksdD*
         (*ksd* ⇒ keto-steroid-degradation)
   (vii) IMMEDIATE SOURCE:
      (A) LIBRARY: --
   (viii) POSITION IN GENOME:
      (B) MAP POSITION: from 1 to 1689 coding region
      (C) UNITS:
   (xi) SEQUENCE DESCRIPTION: SEQ ID NO. 10:
   atgaaatggg atgcaagtta tgatgtagtt gtagtaggct ctggagctgc gggattgaca     60
   gcaggtttaa cagcaaagtt acaaggtttg aaatcattag taattgaaaa aacggatcgc    120
   tatggtggtg cctctgctat ttcaggcggt gccttatgga ttccgaataa tcatgttatt    180
   aaaggtgcag gtgttccaga tacacatgaa cttgcacgcc aatatttaga ttcaacagtt    240
   ggtgatcgag tgcctgaagc tttaaaggaa gcctatatta caagaggccc agaaatgttg    300
   cggtttttat acaataaaac taagcatatg cgtttccaat atgcaaaagg ttactcggac    360
   tactatccag aaaaaccagg gggcttgtct cagggacgtt ccattgaacc actaattttc     420
   gatttaacga aaatgggctc tttagcaaat actatgcgtc gagcaactct atcaactaag    480
   ggctttacaa tgaatagcta tgagtttcat aaagttaata tgataacacg gacgttaaaa    540
   ggtaaaacaa ctgcactgaa attaggcatg cgcctagtaa aatcaaaggt gacaaaaagt    600
   gagccagttg cgttaggtga agctttagta gcacgtttac gactatcgct agcggaggca    660
   aatggtgagc tttggctatc aacggccttt aaagatttta tgatggataa gggtcgagtg    720
   atggggatca ttgtggaacg agatggacaa gagctgcgaa ttgaggcaaa gaaaggtgtt    780
   gttctttcat caggcggctt ttcacacaac caagcacttc gagaacaata tttaccaagc    840
   ccaacgaacg ctgcatggac ttcttcacca gagggacaaa caggtgacgt tatagaacca    900
   ggtgtaaaaa ttggcgctac attagattta atggataaag tgtggggagc gccttctgtt    960
   attgatccac aaggacaacc cttcttccta gtagcggaca ggggcgtacc aaatatgatt   1020
   gttgtagata gcgcaggaca gcgttttgtg aatgaagcgg ctccttatca tgaatttgta   1080
   gataccatgt acgagcatca aaagaccacg caacaggctg ttccttcatg gatagtcatt   1140
   gatgcctcta ctaaaagccg ttatattttt acaggtctgt tcccaggaca agccttccca   1200
   aaaagctggt ttgatcatgg catcgtgaaa agtgcagagt ccattgaaga acttgctaga   1260
   caaatggatg tgctgcctga aagtctaata gagacagtaa atcgttttaa tgactttgcc   1320
   cgaaatggtc atgatgatga tttttatcgt ggtgatagtg cctatgataa ttactatggg   1380
   gacccaacat tgccaaatcc aaatttagca gagatcaaaa aagctccttt ctatgcatta   1440
   cgtatatatc caggcgatat tggcacaaag ggaggcttgg tagtggatga acatgctcgg   1500
   gttattaagg cagatggcga accaatcgaa ggattatatg cttcaggtaa ttgttcagcg   1560
   tcgatcatgg gagaaacgta tcctggtccg ggtgctacga ttgggcctgg tatgacatta   1620
   agctttgtgg cgactacaca tatggctaac accgtaaaaa aagaagaagt accacttgta   1680
   aaaatataa                                                          1689
   a gttgactaag cccttcctat gactgtgata aggaagggct ttcatgtgga   1740
   tgaaatgttc taatattttt ttgctaagaa tatagtggct acaacatgta tggcgatgat   1800
   aatggaaaaa aggagcgata tagtaaattg cttacgtata aacttatcac gactattgaa   1860
   gcattagagc cctatcgaag tact                                         1884
(2) INFORMATION FOR SEQ ID NO: 11:
   (i) SEQUENCE CHARACTERISTICS:
      (A) LENGTH: 562 amino acids
      (B) TYPE: peptide
      (C) STRANDEDNESS: single
      (D) TOPOLOGY: linear
   (ii) MOLECULE TYPE: peptide
   (vi) ORIGINAL SOURCE:
      (A) ORGANISM: *Bacillus sphaericus* ATCC 13805
      (C) INDIVIDUAL ISOLATE: 3-Ketosteroid-Δ¹-Dehydrogenase KsdD
         (Ksd ⇒ Keto-_steroid-_degradation)
   (xi) SEQUENCE DESCRIPTION: SEQ ID NO. 11:
(2) INFORMATION FOR SEQ ID NO: 12:
   (i) SEQUENCE CHARACTERISTICS:
      (A) LENGTH: 1539 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (A) ORGANISM: *Brevibacterium maris* ATCC 2111
      (C) INDIVIDUAL ISOLATE: 3-Ketosteroid-Δ¹-Dehydrogenase-Gen *ksdD*
         (*ksd* ⇒ keto-steroid-degradation)
   (vii) IMMEDIATE SOURCE:
      (A) LIBRARY: --
   (viii) POSITION IN GENOME:
      (B) MAP POSITION: from 1 to 1539 coding region
      (C) UNITS:
   (xi) SEQUENCE DESCRIPTION: SEQ ID NO. 12:
   atggtcaact ggaacgaaga atgtgacgtg ttggtggccg ggtcgggcgc cggtggcgtc    60
   accggcgcgt acaccgcggc tcgcgagggc ctcgacgtga tcctggtcga ggcgacggac   120
   aagttcggcg gcaccaccgc gtactccggt ggcggcgggt tctggttccc ggccaacccg   180
   gtgctcaagc gcgccggcac cgacgacacg atcgaggacg cgctcgagta ctaccacgcc   240
   gtcgtcggcg accggacccc gcgcgagctg caggacacct acgtcaaggg cggcgctccg   300
   ctggtcgagt acctcgagca ggacgagaac ctcaagttcg agatgctgcc gtggcccgac   360
   tactacggca agatgccgaa ggcccgcaac gacggccagc gccacacgat gccgacgccg   420
   ctgccgatct ccgaggtcgg tgacctgcac aagctcgtcc gcggaccgct cgacttcgac   480
   cggctcggcg ccgacctgcc cgagatgctg atcggcggcc gcgcgctcgt cggtcgcttc   540
   ctcaaggcga tcggcaacta cccgaacgcg aagctgaacc tcaacacccc gctcgtcgag   600
   ctggtggtcg aggacggcgc cgtcgtcggc gcgctcgtcg agcgtgacgg cgagcaggtc   660
   gcgatccgcg cccgcaaggg cgtcatcctg gcggccggcg gcttcgaggg caacgacgag   720
   ctgcgccaga agtacggcgt ccccggtgtc gcgcgcgaca cgatgggtcc gtggggcaac   780
   gtcggccagg cgcaccaggc cggcatcgcc gtcggtgccg acaccgacct gatggaccag   840
   gcgtggtggt cgccgggcct gacccacccg gacggacgtt ccgcgttcgc gctgtgcttc   900
   accggcggca tcttcgtcaa cgacgacggc aagcgcttcg tcaacgagta cgcgccgtac   960
   gaccgcctcg gccgcgacat catcgcgggc atggaggacg gctcggtcac gctgccgtac  1020
   tggatgatct acgacgacaa gcagggccag cggccgccga tcgcggccac caacgtctcg  1080
   atggtcgaga ccgagaagta cgtcgacgcc ggcctgtggc acaccgccga cacgctcgag  1140
   gagctggccg gaaagatcgg tgtcccggcg gagaacctgc tggcaacggt ggagcggttc  1200
   aacgcgatgg ccgccaacga cgtcgacgag gacttcggtc gcggcgacga ggcgtacgac  1260
   cgggcgttca ccggcggcgg cccggcgctg atcccgatcg agcagggtcc gttccacgct  1320
   gccgcgttcg gcatctccga cctcggcacc aagggcggtc tgcgtaccga caccgcggcg  1380
   cgggtgctcg acacctcggg caacccgatc cccggtctgt acgcggccgg caacaccatg  1440
   gcggccccga gcggcaccac ctaccccggt ggcggtaacc cgatcggcac ctccatgctg  1500
   ttcagccaca tcgccgcgat gaacatcgcc ggcaagtag                         1539
(2) INFORMATION FOR SEQ ID NO: 13:
   (i) SEQUENCE CHARACTERISTICS:
      (A) LENGTH: 512 amino acids
      (B) TYPE: peptide
      (C) STRANDEDNESS: single
      (D) TOPOLOGY: linear
   (ii) MOLECULE TYPE: peptide
   (vi) ORIGINAL SOURCE:
      (A) ORGANISM: *Brevibacterium maris* ATCC 2111
      (C) INDIVIDUAL ISOLATE: 3-Ketosteroid-Δ¹-Dehydrogenase KsdD
         (Ksd ⇒ Keto-steroid-degradation)
   (xi) SEQUENCE DESCRIPTION: SEQ ID NO. 13:
(2) INFORMATION FOR SEQ ID NO: 14:
   (i) SEQUENCE CHARACTERISTICS:
      (A) LENGTH: 515 amino acids
      (B) TYPE: peptide
      (C) STRANDEDNESS: single
      (D) TOPOLOGY: linear
   (ii) MOLECULE TYPE: peptide
   (vi) ORIGINAL SOURCE:
      (A) ORGANISM: *Arthrobacter simplex*
      (C) INDIVIDUAL ISOLATE: 3-Ketosteroid-Δ¹-Dehydrogenase KsdD
         (Ksd ⇒ Keto-steroid-degradation)
   (vii) IMMEDIATE SOURCE:
      (A) LIBRARY: EMBL/ GenBank, AC:D37969
   (xi) SEQUENCE DESCRIPTION: SEQ ID NO. 14:
(2) INFORMATION FOR SEQ ID NO: 15:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (C) INDIVIDUAL ISOLATE: Primer 2048
   (vii) IMMEDIATE SOURCE:
      (A) LIBRARY:
   (xi) SEQUENCE DESCRIPTION: SEQ ID NO. 15:
      GAA TRY GAT NTW NTW GTW GYW GGW WSW GG
      mit N ≡ GATC, R ≡ GA, S ≡ GC, W ≡ AT, Y ≡ TC
(2) INFORMATION FOR SEQ ID NO: 16:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (C) INDIVIDUAL ISOLATE: Primer 2054
   (vii) IMMEDIATE SOURCE:
      (A) LIBRARY: --
   (xi) SEQUENCE DESCRIPTION: SEQ ID NO. 16:
      NAR NCC NCC YTT NGT NCC
      mit N ≡ GATC, R≡ GA, S ≡ GC, W ≡ AT, Y ≡ TC
(2) INFORMATION FOR SEQ ID NO: 17:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (C) INDIVIDUAL ISOLATE: Primer 2089ₘᵤₜ
   (vii) IMMEDIATE SOURCE:
      (A) LIBRARY: Seq. ID No. 9
   (viii) POSITION IN GENOME:
      (B) MAP POSITION: from -29 to -91 promoter region
      (C) UNITS:
   (xi) SEQUENCE DESCRIPTION: SEQ ID NO. 17:
      CCA TCG ATG AAT CTG GTC TTC CTA TTA AAA ATT ATA GAA TTA AAC TAA
      TAT TCT GTC AAT TTT TCC_{-29 bis -91}
      **mit N ≡ GATC, R ≡ GA, S ≡ GC, W ≡ AT, Y ≡ TC**
(2) INFORMATION FOR SEQ ID NO: 18:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (C) INDIVIDUAL ISOLATE: Primer 2090
   (vii) IMMEDIATE SOURCE:
      (A) LIBRARY: Seq. ID No. 9
   (viii) POSITION IN GENOME:
      (B) MAP POSITION: from -258 to -284
      (C) UNITS:
   (xi) SEQUENCE DESCRIPTION: SEQ ID NO. 18:
      CAT GAC AAA ATT ATT TGA TTT AAT CAC₋₂₅₈ bis ₋₂₈₄
      mit N ≡ GATC, R ≡ GA, S ≡ GC, W ≡ AT, Y ≡ TC
(2) INFORMATION FOR SEQ ID NO: 19:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (C) INDIVIDUAL ISOLATE: Oligonukleotid 2091*_{parS}*
   (vii) IMMEDIATE SOURCE:
      (A) LIBRARY: Lin DC and Grossman AD (1998, Cell 92: 675-685)
   (xi) SEQUENCE DESCRIPTION: SEQ ID NO. 19:
      GAT CCT GTT CCA CGT GAA ACA G
(2) INFORMATION FOR SEQ ID NO: 20:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: nucleic acid
   (iii) HYPOTHETICAL: No
   (iii) ANTI-SENSE: No
   (vi) ORIGINAL SOURCE:
      (C) INDIVIDUAL ISOLATE: Oligonukleotid 2092*_{parS}*
   (vii) IMMEDIATE SOURCE:
      (A) LIBRARY: Lin DC and Grossman AD (1998, Cell 92: 675-685)
   (xi) SEQUENCE DESCRIPTION: SEQ ID NO. 20:
      GAT CCT GTT TCA CGT GGA ACA G

### SEQUENCE LISTING

<110> SCHERING AKTIENGESELLSCHAFT
<120> verfahren zur Überexpression von Dehydrogenasen
<130> 52287AWO
<140> PCT/EP03/00855
   <141> 2003-01-28
<150> DE10204798.7
   <151> 2002-02-01
<160> 20
<170> PatentIn version 3.1
<210> 1
   <211> 1548
   <212> DNA
   <213> Arthrobacter sp. ATCC 6946
<400> 1
   atggactggg cagaggagta cgacgtactg gtggcgggct ccggcgccgg cggcatggcc      60
   gggacctaca ccgcggcccg cgaggggctc agcgtgtgcc tggtcgaggc cggggacaag     120
   ttcggcggga cgaccgccta ctccggcggc ggtggggcct ggttccccgc gaacccggtg     180
   ctgctgcggg cgggcaccga cgacacgatc gaggacgctc tcgagtacta ccgagcggtc     240
   gtcggcgacc gcacccccgc ggacctgcag gagacctacg tccgcggcgg cgccggcctg      300
   gtcgcctacc tcgaggagga cgaccacttc tccttcgagt cctacccgtg gccggactac     360
   ttcggcgacg cccccaaggc ccgtcgcgac ggccagcggc acatcatccc gacgccgctg     420
   ccggtgccct ccgcacccga gctgcgcgag gtggtccgcg ggccgctcga caacgaccgg     480
   ctcggcacgc cgcagcccga cgacctgttc atcggcggac gggcgctcgt cgcccgcttc     540
   ctgaccgcgc tcgcgaccta cccccacgcc acgctcgtgc gcgagaccgc actggccgag     600
   ctcgtcgtcg aggacggcgt cgtggtcggc gcgatcgtcg agaccgacgg cgtccgccgc     660
   gcgatccggg cccgccgcgg cgtcctcctg gccgcgggcg gcttcgaggc caatgacgag     720
   ctccgccaga agtacggcgt ccccggcgtc gcgcgcgaca cgatgggccc gccgaccaac     780
   gtcggcgccg cgcaccaggc cgcgatcgcg gtcggcgccg acaccgacct gatgggcgag     840
   gcctggtggt cccccgggct gacccacccc gacggacgat cggcgttcgc gctctggttc     900
   accggcggca tcttcgtcga cggcgccggc cggcgcttcg tcaacgagtc ggcgccgtac     960
   gaccggctcg gccgcgccgt catcgaccac ctcaccgagg gcggcgtcac cccgcggtac    1020
   tggatggtct acgaccacaa ggagggctcg atccccccgg tgcgcgccac caacgtctcg     1080
   atggtcgacg aggagcagta cgtcgccgcg ggcctgtggc acaccgccga cacgctgccc    1140
   gagctggccg cgctgatcgg cgtccccgcc gacgcgctgg tcgccacggt cgcgcgcttc    1200
   aacgagctcg tcgccgacgg gtacgacgcg gacttcggcc gcggcggcga ggcctacgac    1260
   cggttcttct ccggcggcga gccgccgctg gtgagcatcg acgaggggcc gttccacgcg    1320
   gccgccttcg gcatctccga cctcggcacc aagggcgggc tgcgcaccga cacgtccgcg    1380
   cgcgtgctga ccgcggacgg cacgccgatc gggggcctct acgcagccgg caatacgatg    1440
   gcggcgccga gcggcaccac ctacccgggc ggtggcaacc cgatcgggac aagcatgctc    1500
   ttcagccacc tcgccgtgcg gcacatgggc accgaggacg cgcgatga                 1548
<210> 2
   <211> 32
   <212> DNA
   <213> INDIVIDUAL ISOLATE. Primer 2026
<400> 2
   gatcacgatg gactgggcag aggagtacga cg 32
<210> 3
   <211> 33
   <212> DNA
   <213> INDIVIDUAL ISOLATE: Primer 2017
<400> 3
   gacgccgtac tctggcggag ctcgtcattg gcc 33
<210> 4
   <211> 42
   <212> DNA
<213> INDIVIDUAL ISOLATION: Primer 2026
<400> 4
   cgggatccat ggactgggca gaggagtacg acgtactggt gg 42
<210> 5
   <211> 42
   <212> DNA
   <213> INDIVIDUAL ISOLATION: Primer
<400> 5
   cggaattctc atcgcgcgtc ctcggtgccc atgtgccgca cg 42
<210> 6
   <211> 19
   <212> DNA
   <213> INDIVIDUAL ISOLATION: Primer 2032
<400> 6
   cgatcgtcga gaccgacgg 19
<210> 7
   <211> 27
   <212> DNA
   <213> INDIVIDUAL ISOLATION: Primer 2055
<400> 7
   gcagcaccgg gttcgcgggg aaccagg 27
<210> 8
   <211> 3630
   <212> DNA
   <213> Arthrobacter sp. ATCC 6946
<400> 8
   ctgcaggagc tcggcctggt cgagcgggcc gcggacacct tcgaccggcg caccacgctg 60
   gtccgctgct cgcgccgcgg cgtcgcccag gtacgccggc tcgcggccgc ccagcgcgcc 120
   gacctagccg ccgcgctcgg tccggtcgac ccggccgacc gggaccgctg gacggtgctc 180
   gtggagcgct acgtgcgggc tctcgaggcc cgcgggctca tctccgagct gtgactcgcc 240
   ggtaagttca gagaacatta tgtgcaaacg gtccagtaaa actagccgtt cggcaagtag 300
   attggtgacc catcgcattc tgtgtttccg caggtcagag gcacagtttc ggaggtgacc 360
   gcagtcccgg tgaccgggag tgccgattca cggcggaaac ctcaccgaaa aatatgtgcg 420
   ttcgatccac ttgatttgcc ctgtgtcagt gctcacactc gacgggaggc cgcactcccg 480
   aggagcaccc gcatgaccgt caccgcactg cccacgacca cgcccgccgg ctccggcgca 540
   cccgccctgg accccgacga ccgccgcacg cccctgggcg tcgtgggccg ggtgacccgg 600
   atcctcaacg ccttcagcga gtcccccgac cgcctcatgc tcgaggacgt gatggcgctg 660
   accggcctgc cccggtcgac cgccttccgg atcctcggcc agctcatcga cgaggggtgg 720
   gtcgagcacg acacccgcgg ctaccggctc gggccgcacg cgcccacgct caccggccgg 780
   cccggcgagc accaggaggt gcgggtcgcc gcgtcgccgt acctcaacga gctgcacgcc 840
   ctcaccggcg cggtcgccca cctctcggtg ctcgagggcg accgggtcca ctacctcgac 900
   aagatcggcg gctccgcggc tcgcgccgtc ccctcgcggg tcggcgcccg gctgctcgcc 960
   tccgacaccg tcagcggccg cgcgctgctc gcctgccgct cccccgagta cgtcgacgac 1020
   gtcctcggcc cgcggctgcc cgcgccccgg ctcgccctgc tccaccgcga cctcgccgcc 1080
   gcccgccagc gccgcggcgt cgtgcacgcc ccggccgacc cgaccaccgg catcgcctcg 1140
   atcgccgcac ccgtcctcgg cccgcacgga gccgtcgccg cgatctcgct ggccctgccc 1200
   ggcgagctgc cgcccgcccg gctcgcaccc ctgctgctca accaggccca ccggatcgcc 1260
   ggcgtcctgt tcccccagcg ccgcctgcac ggacgatcct ggctgcgctg atcccgcccc 1320
   cgcccggaga ctcccgcagg acgggagaac ccaccggggc acccggggcc gctgcctagc 1380
   gtcgccgcca cgacgccgga ggtcggcgtc ggtcaacccg gcgagaggat cacgatggac 1440
   tgggcagagg agtacgacgt actggtggcg ggctccggcg ccggcggcat ggccgggacc 1500
   tacaccgcgg cccgcgaggg gctcagcgtg tgcctggtcg aggccgggga caagttcggc 1560
   gggacgaccg cctactccgg cggcggtggg gcctggttcc ccgcgaaccc ggtgctgctg 1620
   cgggcgggca ccgacgacac gatcgaggac gctctcgagt actaccgagc ggtcgtcggc 1680
   gaccgcaccc ccgcggacct gcaggagacc tacgtccgcg gcggcgccgg cctggtcgcc 1740
   tacctcgagg aggacgacca cttctccttc gagtcctacc cgtggccgga ctacttcggc 1800
   gacgccccca aggcccgtcg cgacggccag cggcacatca tcccgacgcc gctgccggtg 1860
   ccctccgcac ccgagctgcg cgaggtggtc cgcgggccgc tcgacaacga ccggctcggc 1920
   acgccgcagc ccgacgacct gttcatcggc ggacgggcgc tcgtcgcccg cttcctgacc 1980
   gcgctcgcga cctaccccca cgccacgctc gtgcgcgaga ccgcactggc cgagctcgtc 2040
   gtcgaggacg gcgtcgtggt cggcgcgatc gtcgagaccg acggcgtccg ccgcgcgatc 2100
   cgggcccgcc gcggcgtcct cctggccgcg ggcggcttcg aggccaatga cgagctccgc 2160
   cagaagtacg gcgtccccgg cgtcgcgcgc gacacgatgg gcccgccgac caacgtcggc 2220
   gccgcgcacc aggccgcgat cgcggtcggc gccgacaccg acctgatggg cgaggcctgg 2280
   tggtcccccg ggctgaccca ccccgacgga cgatcggcgt tcgcgctctg gttcaccggc 2340
   ggcatcttcg tcgacggcgc cggccggcgc ttcgtcaacg agtcggcgcc gtacgaccgg 2400
   ctcggccgcg ccgtcatcga ccacctcacc gagggcggcg tcaccccgcg gtactggatg 2460
   gtctacgacc acaaggaggg ctcgatcccc ccggtgcgcg ccaccaacgt ctcgatggtc 2520
   gacgaggagc agtacgtcgc cgcgggcctg tggcacaccg ccgacacgct gcccgagctg 2580
   gccgcgctga tcggcgtccc cgccgacgcg ctggtcgcca cggtcgcgcg cttcaacgag 2640
   ctcgtcgccg acgggtacga cgcggacttc ggccgcggcg gcgaggccta cgaccggttc 2700
   ttctccggcg gcgagccgcc gctggtgagc atcgacgagg ggccgttcca cgcggccgcc 2760
   ttcggcatct ccgacctcgg caccaagggc gggctgcgca ccgacacgtc cgcgcgcgtg 2820
   ctgaccgcgg acggcacgcc gatcgggggc ctctacgcag ccggcaatac gatggcggcg 2880
   ccgagcggca ccacctaccc gggcggtggc aacccgatcg ggacaagcat gctcttcagc 2940
   cacctcgccg tgcggcacat gggcaccgag gacgcgcgat gagcgccgag gtgaaggccg 3000
   ccgtggcgcg ctacctcgat gctgtcgccg gcggctcgcc ggccgcgatc gccgcgctct 3060
   acgcccccga cgccacgctc gaggaccccg tcggcgccga cctcgtccgc ggccgcgcgg 3120
   cgatcgaaga gttctacggc gccctcgccg gcgcgaaggt cagcaccgag ctgctcgccg 3180
   tccgcgccgt cgcgggccac gccgcgttct cgttccgggt caccaccgac gccggcgacc 3240
   agcagtacgt cgtcgagccg atcgacgtga tgacgttcga cgcggacggc cagatcacgt 3300
   ccatgcgggc gttctgggcg cccggggaca tggtcgtcac gccggcctga cggtcccgct 3360
   gtaacacgct gtccaccgcg cttcccggcg gttgtcgacg cgctctcggc gtgtcgcacg 3420
   gcgtgtcgcg ccgtggacag cgtgttacag cggcgggggc cgtcaggcgg tggccgcgtg 3480
   ggtggcgacg atgtggccga agaccagacc ctggccgatg gtcgcgccgg cccccgggta 3540
   gctgcgcccg aagacgttgc ccgcggtgtt gccgatcgcg tagagcccct cgatcgggct 3600
   gccgtcggcg cgcagcggac ggccgagctc 3630
<210> 9
   <211> 284
   <212> DNA
   <213> Bacillus sphaericus ATCC 13805
<400> 9
   catgacaaaa ttatttgatt taatcactgc aggaaagttt gatccgactg acataattac 60
   acataagcta ccattagaag aagcaagtaa agcctatcaa ctatttagta accgtgaaga 120
   taactgtatt aaagtgattt taaaacctta aagggagcgt cgacgctcct ttttttgtgt 180
   gtaatgttgg gatggaaaaa ttgactgaat attagtttaa ttctatactt tttaatagga 240
   agaccagatt catcgattta gctcattaag ggaggaatgg ttga 284
<210> 10
   <211> 1884
   <212> DNA
   <213> Bacillus sphaericus ATCC 13805
<400> 10
   atgaaatggg atgcaagtta tgatgtagtt gtagtaggct ctggagctgc gggattgaca 60
   gcaggtttaa cagcaaagtt acaaggtttg aaatcattag taattgaaaa aacggatcgc 120
   tatggtggtg cctctgctat ttcaggcggt gccttatgga ttccgaataa tcatgttatt 180
   aaaggtgcag gtgttccaga tacacatgaa cttgcacgcc aatatttaga ttcaacagtt 240
   ggtgatcgag tgcctgaagc tttaaaggaa gcctatatta caagaggccc agaaatgttg 300
   cggtttttat acaataaaac taagcatatg cgtttccaat atgcaaaagg ttactcggac 360
   tactatccag aaaaaccagg gggcttgtct cagggacgtt ccattgaacc actaattttc 420
   gatttaacga aaatgggctc tttagcaaat actatgcgtc gagcaactct atcaactaag 480
   ggctttacaa tgaatagcta tgagtttcat aaagttaata tgataacacg gacgttaaaa 540
   ggtaaaacaa ctgcactgaa attaggcatg cgcctagtaa aatcaaaggt gacaaaaagt 600
   gagccagttg cgttaggtga agctttagta gcacgtttac gactatcgct agcggaggca 660
   aatggtgagc tttggctatc aacggccttt aaagatttta tgatggataa gggtcgagtg 720
   atggggatca ttgtggaacg agatggacaa gagctgcgaa ttgaggcaaa gaaaggtgtt 780
   gttctttcat caggcggctt ttcacacaac caagcacttc gagaacaata tttaccaagc 840
   ccaacgaacg ctgcatggac ttcttcacca gagggacaaa caggtgacgt tatagaacca 900
   ggtgtaaaaa ttggcgctac attagattta atggataaag tgtggggagc gccttctgtt 960
   attgatccac aaggacaacc cttcttccta gtagcggaca ggggcgtacc aaatatgatt 1020
   gttgtagata gcgcaggaca gcgttttgtg aatgaagcgg ctccttatca tgaatttgta 1080
   gataccatgt acgagcatca aaagaccacg caacaggctg ttccttcatg gatagtcatt 1140
   gatgcctcta ctaaaagccg ttatattttt acaggtctgt tcccaggaca agccttccca 1200
   aaaagctggt ttgatcatgg catcgtgaaa agtgcagagt ccattgaaga acttgctaga 1260
   caaatggatg tgctgcctga aagtctaata gagacagtaa atcgttttaa tgactttgcc 1320
   cgaaatggtc atgatgatga tttttatcgt ggtgatagtg cctatgataa ttactatggg 1380
   gacccaacat tgccaaatcc aaatttagca gagatcaaaa aagctccttt ctatgcatta 1440
   cgtatatatc caggcgatat tggcacaaag ggaggcttgg tagtggatga acatgctcgg 1500
   gttattaagg cagatggcga accaatcgaa ggattatatg cttcaggtaa ttgttcagcg 1560
   tcgatcatgg gagaaacgta tcctggtccg ggtgctacga ttgggcctgg tatgacatta 1620
   agctttgtgg cgactacaca tatggctaac accgtaaaaa aagaagaagt accacttgta 1680
   aaaatataaa gttgactaag cccttcctat gactgtgata aggaagggct ttcatgtgga 1740
   tgaaatgttc taatattttt ttgctaagaa tatagtggct acaacatgta tggcgatgat 1800
   aatggaaaaa aggagcgata tagtaaattg cttacgtata aacttatcac gactattgaa 1860
   gcattagagc cctatcgaag tact 1884
<210> 11
   <211> 562
   <212> PRT
   <213> Bacillus sphaericus ATCC 13805
<400> 11
   Met Lys Trp Asp Ala Ser Tyr Asp Val Val Val Val Gly Ser Gly Ala
   1               5                   10                  15
   Ala Gly Leu Thr Ala Gly Leu Thr Ala Lys Leu Gln Gly Leu Lys Ser
               20                  25                  30
   Leu Val Ile Glu Lys Thr Asp Arg Tyr Gly Gly Ala Ser Ala Ile Ser
           35                  40                  45
   Gly Gly Ala Leu Trp Ile Pro Asn Asn His Val Ile Lys Gly Ala Gly
       50                  55                  60
   Val Pro Asp Thr His Glu Leu Ala Arg Gln Tyr Leu Asp Ser Thr Val
   65                  70                  75                  80
   Gly Asp Arg Val Pro Glu Ala Leu Lys Glu Ala Tyr Ile Thr Arg Gly
                   85                  90                  95
   Pro Glu Met Leu Arg Phe Leu Tyr Asn Lys Thr Lys His Met Arg Phe
               100                 105                 110
   Gln Tyr Ala Lys Gly Tyr Ser Asp Tyr Tyr Pro Glu Lys Pro Gly Gly
           115                 120                 125
   Leu Ser Gln Gly Arg Ser Ile Glu Pro Leu Ile Phe Asp Leu Thr Lys
       130                 135                 140
   Met Gly Ser Leu Ala Asn Thr Met Arg Arg Ala Thr Leu Ser Thr Lys
   145                 150                 155                 160
   Gly Phe Thr Met Asn Ser Tyr Glu Phe His Lys Val Asn Met Ile Thr
                   165                 170                 175
   Arg Thr Leu Lys Gly Lys Thr Thr Ala Leu Lys Leu Gly Met Arg Leu
               180                 185                 190
   Val Lys Ser Lys Val Thr Lys Ser Glu Pro Val Ala Leu Gly Glu Ala
   195 200 205
   Leu Val Ala Arg Leu Arg Leu Ser Leu Ala Glu Ala Asn Gly Glu Leu
   210 215 220
   Trp Leu Ser Thr Ala Phe Lys Asp Phe Met Met Asp Lys Gly Arg Val
   225 230 235 240
   Met Gly Ile Ile Val Glu Arg Asp Gly Gln Glu Leu Arg Ile Glu Ala
   245 250 255
   Lys Lys Gly Val Val Leu Ser Ser Gly Gly Phe Ser His Asn Gln Ala
   260 265 270
   Leu Arg Glu Gln Tyr Leu Pro Ser Pro Thr Asn Ala Ala Trp Thr Ser
   275 280 285
   Ser Pro Glu Gly Gln Thr Gly Asp Val Ile Glu Pro Gly Val Lys Ile
   290 295 300
   Gly Ala Thr Leu Asp Leu Met Asp Lys Val Trp Gly Ala Pro Ser Val
   305 310 315 320
   Ile Asp Pro Gln Gly Gln Pro Phe Phe Leu Val Ala Asp Arg Gly Val
   325 330 335
   Pro Asn Met Ile Val Val Asp Ser Ala Gly Gln Arg Phe Glu Asn Glu
   340 345 350
   Ala Ala Pro Tyr His Glu Phe Val Asp Thr Met Tyr Glu His Gln Lys
   355 360 365
   Thr Thr Gln Gln Ala Val Pro Ser Trp Ile Val Ile Asp Ala Ser Thr
   370 375 380
   Lys Ser Arg Tyr Ile Phe Thr Gly Leu Phe Pro Gly Gln Ala Phe Pro
   385 390 395 400
   Lys Ser Trp Phe Asp His Gly Ile Val Lys Ser Ala Glu Ser Ile Glu
   405 410 415
   Glu Leu Ala Arg Gln Met Asp Val Leu Leu Glu Ser Leu Ile Glu Thr
   420 425 430
   Val Asn Arg Phe Asn Asp Phe Ala Arg Asn Gly His Asp Asp Asp Phe
   435 440 445
   Tyr Arg Gly Asp Ser Val Tyr Asp Asn Tyr Tyr Gly Asp Pro Thr Leu
   450 455 460
   Pro Asn Pro Asn Leu Ala Glu Ile Lys Lys Ala Pro Phe Tyr Ala Leu
   465 470 475 480
   Arg Ile Tyr Pro Gly Asp Ile Gly Thr Lys Gly Gly Leu Val Asp Glu
   485 490 495
   His Ala Arg Val Ile Lys Ala Asp Gly Glu Pro Ile Glu Gly Leu Tyr
   500 505 510
   Ala Ser Gly Asn Cys Ser Ala Ser Ile Met Gly Glu Thr Tyr Pro Gly
   515 520 525
   Pro Gly Ala Thr Ile Gly Pro Gly Met Thr Leu Ser Phe Val Ala Ala
   530 535 540
   Thr Thr His Met Ala Asn Thr Val Lys Lys Glu Glu Val Pro Leu Val
   545 550 555 560
   Lys Ile
<210> 12
   <211> 1539
   <212> DNA
   <213> Brevibacterium maris ATCC 2111
<400> 12
   atggtcaact ggaacgaaga atgtgacgtg ttggtggccg ggtcgggcgc cggtggcgtc 60
   accggcgcgt acaccgcggc tcgcgagggc ctcgacgtga tcctggtcga ggcgacggac 120
   aagttcggcg gcaccaccgc gtactccggt ggcggcgggt tctggttccc ggccaacccg 180
   gtgctcaagc gcgccggcac cgacgacacg atcgaggacg cgctcgagta ctaccacgcc 240
   gtcgtcggcg accggacccc gcgcgagctg caggacacct acgtcaaggg cggcgctccg 300
   ctggtcgagt acctcgagca ggacgagaac ctcaagttcg agatgctgcc gtggcccgac 360
   tactacggca agatgccgaa ggcccgcaac gacggccagc gccacacgat gccgacgccg 420
   ctgccgatct ccgaggtcgg tgacctgcac aagctcgtcc gcggaccgct cgacttcgac 480
   cggctcggcg ccgacctgcc cgagatgctg atcggcggcc gcgcgctcgt cggtcgcttc 540
   ctcaaggcga tcggcaacta cccgaacgcg aagctgaacc tcaacacccc gctcgtcgag 600
   ctggtggtcg aggacggcgc cgtcgtcggc gcgctcgtcg agcgtgacgg cgagcaggtc 660
   gcgatccgcg cccgcaaggg cgtcatcctg gcggccggcg gcttcgaggg caacgacgag 720
   ctgcgccaga agtacggcgt ccccggtgtc gcgcgcgaca cgatgggtcc gtggggcaac 780
   gtcggccagg cgcaccaggc cggcatcgcc gtcggtgccg acaccgacct gatggaccag 840
   gcgtggtggt cgccgggcct gacccacccg gacggacgtt ccgcgttcgc gctgtgcttc 900
   accggcggca tcttcgtcaa cgacgacggc aagcgcttcg tcaacgagta cgcgccgtac     960
   gaccgcctcg gccgcgacat catcgcgggc atggaggacg gctcggtcac gctgccgtac    1020
   tggatgatct acgacgacaa gcagggccag cggccgccga tcgcggccac caacgtctcg    1080
   atggtcgaga ccgagaagta cgtcgacgcc ggcctgtggc acaccgccga cacgctcgag    1140
   gagctggccg gaaagatcgg tgtcccggcg gagaacctgc tggcaacggt ggagcggttc    1200
   aacgcgatgg ccgccaacga cgtcgacgag gacttcggtc gcggcgacga ggcgtacgac    1260
   cgggcgttca ccggcggcgg cccggcgctg atcccgatcg agcagggtcc gttccacgct    1320
   gccgcgttcg gcatctccga cctcggcacc aagggcggtc tgcgtaccga caccgcggcg    1380
   cgggtgctcg acacctcggg caacccgatc cccggtctgt acgcggccgg caacaccatg    1440
   gcggccccga gcggcaccac ctaccccggt ggcggtaacc cgatcggcac ctccatgctg    1500
   ttcagccaca tcgccgcgat gaacatcgcc ggcaagtag                           1539
<210> 13
   <211> 512
   <212> PRT
   <213> Brevibacterium maris ATCC 2111
<400> 13
   Met Val Asn Trp Asn Glu Glu Cys Asp Val Leu Val Ala Gly Ser Gly
   1               5                   10                  15
   Ala Gly Gly Val Thr Gly Ala Tyr Thr Ala Ala Arg Glu Gly Leu Asp
               20                  25                  30
   Val Ile Leu Val Glu Ala Thr Asp Lys Phe Gly Gly Thr Thr Ala Tyr
           35                  40                  45
   Ser Gly Gly Gly Gly Phe Trp Phe Pro Ala Asn Pro Val Leu Lys Arg
       50                  55                  60
   Ala Gly Thr Asp Asp Thr Ile Glu Asp Ala Leu Glu Tyr Tyr His Ala
   65                  70                  75                  80
   Val Val Gly Asp Arg Thr Pro Arg Glu Leu Gln Asp Thr Tyr Val Lys
                   85                  90                  95
   Gly Gly Ala Pro Leu Val Glu Tyr Leu Glu Gln Asp Glu Asn Leu Lys
               100                 105                 110
   Phe Glu Met Leu Pro Trp Pro Asp Tyr Tyr Gly Lys Met Pro Lys Ala
           115                 120                 125
   Arg Asn Asp Gly Gln Arg His Thr Met Pro Thr Pro Leu Pro Ile Ser
   130 135 140
   Glu Val Gly Asp Leu His Lys Leu Val Arg Gly Pro Leu Asp Phe Asp
   145 150 155 160
   Arg Leu Gly Ala Asp Leu Pro Glu Met Leu Ile Gly Gly Arg Ala Leu
   165 170 175
   Val Gly Arg Phe Leu Lys Ala Ile Gly Asn Tyr Pro Asn Ala Lys Leu
   180 185 190
   Asn Leu Asn Thr Pro Leu Val Glu Leu Val Val Glu Asp Gly Ala Val
   195 200 205
   Val Gly Ala Leu Val Glu Arg Asp Gly Glu Gln Val Ala Ile Arg Ala
   210 215 220
   Arg Lys Gly Val Ile Leu Ala Ala Gly Gly Phe Glu Gly Asn Asp Glu
   225 230 235 240
   Leu Arg Gln Lys Tyr Gly Val Pro Gly Val Ala Arg Asp Thr Met Gly
   245 250 255
   Pro Trp Gly Asn Val Gly Gln Ala His Gln Ala Gly Ile Ala Val Gly
   260 265 270
   Ala Asp Thr Asp Leu Met Asp Gln Ala Trp Trp Ser Pro Gly Leu Thr
   275 280 285
   His Pro Asp Gly Arg Ser Ala Phe Ala Leu Cys Phe Thr Gly Gly Ile
   290 295 300
   Phe Val Asn Asp Asp Gly Lys Arg Phe Val Asn Glu Tyr Ala Pro Tyr
   305 310 315 320
   Asp Arg Leu Gly Arg Asp Ile Ile Ala Gly Met Glu Asp Gly Ser Val
   325 330 335
   Thr Leu Pro Tyr Trp Met Ile Tyr Asp Asp Lys Gln Gly Gln Arg Pro
   340 345 350
   Pro Ile Ala Ala Thr Asn Val Ser Met Val Glu Thr Glu Lys Tyr Val
   355 360 365
   Asp Ala Gly Leu Trp His Thr Ala Asp Thr Leu Glu Glu Leu Ala Gly
   370 375 380
   Lys Ile Gly Val Pro Ala Glu Asn Leu Leu Ala Thr Val Glu Arg Phe
   385 390 395 400
   Asn Ala Met Ala Ala Asn Asp Val Asp Glu Asp Phe Gly Arg Gly Asp
   405 410 415
   Glu Ala Tyr Asp Arg Ala Phe Thr Gly Gly Gly Pro Ala Leu Ile Pro
   420 425 430
   Ile Glu Gln Gly Pro Phe His Ala Ala Ala Phe Gly Ile Ser Asp Leu
   435 440 445
   Gly Thr Lys Gly Gly Leu Arg Thr Asp Thr Ala Ala Arg Val Leu Asp
   450 455 460
   Thr Ser Gly Asn Pro Ile Pro Gly Leu Tyr Ala Ala Gly Asn Thr Met
   465 470 475 480
   Ala Ala Pro Ser Gly Thr Thr Tyr Pro Gly Gly Gly Asn Pro Ile Gly
   485 490 495
   Thr Ser Met Leu Phe Ser His Ile Ala Ala Met Asn Ile Ala Gly Lys
   500 505 510
<210> 14
   <211> 515
   <212> PRT
   <213> Arthrobacter sp.
<400> 14
   Met Asp Trp Ala Glu Glu Tyr Asp Val Leu Val Ala Gly Ser Gly Ala
   1 5 10 15
   Gly Gly Met Ala Gly Thr Tyr Thr Ala Ala Arg Glu Gly Leu Ser Val
   20 25 30
   Cys Leu Val Glu Ala Gly Asp Lys Phe Gly Gly Thr Thr Ala Tyr Ser
   35 40 45
   Gly Gly Gly Gly Ala Trp Phe Pro Ala Asn Pro Val Leu Leu Arg Ala
   50 55 60
   Gly Thr Asp Asp Thr Ile Glu Asp Ala Leu Glu Tyr Tyr Arg Ala Val
   65 70 75 80
   Val Gly Asp Arg Thr Pro Ala Asp Leu Gln Glu Thr Tyr Val Arg Gly
   85 90 95
   Gly Ala Gly Leu Val Ala Tyr Leu Glu Glu Asp Asp His Phe Ser Phe
   100 105 110
   Glu Ser Tyr Pro Trp Pro Asp Tyr Phe Gly Asp Ala Pro Lys Ala Arg
           115                 120                 125
   Arg Asp Gly Gln Arg His Ile Ile Pro Thr Pro Leu Pro Val Pro Ser
       130                 135                 140
   Ala Pro Glu Leu Arg Glu Val Val Arg Gly Pro Leu Asp Asn Asp Arg
   145                 150                 155                 160
   Leu Gly Thr Pro Gln Pro Asp Asp Leu Phe Ile Gly Gly Arg Ala Leu
                   165                 170                 175
   Val Ala Arg Phe Leu Thr Ala Leu Ala Thr Tyr Pro His Ala Thr Leu
               180                 185                 190
   Val Arg Glu Thr Ala Leu Ala Glu Leu Val Val Glu Asp Gly Val Val
           195                 200                 205
   Val Gly Ala Ile Val Glu Thr Asp Gly Val Arg Arg Ala Ile Arg Ala
       210                 215                 220
   Arg Arg Gly Val Leu Leu Ala Ala Gly Gly Phe Glu Ala Asn Asp Glu
   225                 230                 235                 240
   Leu Arg Gln Lys Tyr Gly Val Pro Gly Val Ala Arg Asp Thr Met Gly
                   245                 250                 255
   Pro Pro Thr Asn Val Gly Ala Ala His Gln Ala Ala Ile Ala Val Gly
               260                 265                 270
   Ala Asp Thr Asp Leu Met Gly Glu Ala Trp Trp Ser Pro Gly Leu Thr
           275                 280                 285
   His Pro Asp Gly Arg Ser Ala Phe Ala Leu Trp Phe Thr Gly Gly Ile
       290                 295                 300
   Phe Val Asp Gly Ala Gly Arg Arg Phe Val Asn Glu Ser Ala Pro Tyr
   305                 310                 315                 320
   Asp Arg Leu Gly Arg Ala Val Ile Asp His Leu Thr Glu Gly Gly Val
                   325                 330                 335
   Thr Pro Arg Tyr Trp Met Val Tyr Asp His Lys Glu Gly Ser Ile Pro
               340                 345                 350
   Pro Val Arg Ala Thr Asn Val Ser Met Val Asp Glu Glu Gln Tyr Val
           355                 360                 365
   Ala Ala Gly Leu Trp His Thr Ala Asp Thr Leu Pro Glu Leu Ala Ala
       370                 375                 380
   Leu Ile Gly Val Pro Ala Asp Ala Leu Val Ala Thr Val Ala Arg Phe
   385 390 395 400
   Asn Glu Leu Val Ala Asp Gly Tyr Asp Ala Asp Phe Gly Arg Gly Gly
   405 410 415
   Glu Ala Tyr Asp Arg Phe Phe Ser Gly Gly Glu Pro Pro Leu Val Ser
   420 425 430
   Ile Asp Glu Gly Pro Phe His Ala Ala Ala Phe Gly Ile Ser Asp Leu
   435 440 445
   Gly Thr Lys Gly Gly Leu Arg Thr Asp Thr Ser Ala Arg Val Leu Thr
   450 455 460
   Ala Asp Gly Thr Pro Ile Gly Gly Leu Tyr Ala Ala Gly Asn Thr Met
   465 470 475 480
   Ala Ala Pro Ser Gly Thr Thr Tyr Pro Gly Gly Gly Asn Pro Ile Gly
   485 490 495
   Thr Ser Met Leu Phe Ser His Leu Ala Val Arg His Met Gly Thr Glu
   500 505 510
   Asp Ala Arg
   515
<210> 15
   <211> 29
   <212> DNA
   <213> Individual isolate Primer 2048
   gaa try gat ntw ntw gtw gyw ggw wsw gg
<210> 16
   <211> 18
   <212> DNA
   <213> Individual isolate Primer 2054
   nar ncc ncc ytt ngt ncc
<210> 17
   <211> 66
   <212> DNA
   <213> INDUVIDUAL ISOLATE: Primer 2089mut
<400> 17
   ccatcgatga atctggtctt cctattaaaa attatagaat taaactaata ttctgtcaat 60
   ttttcc 66
<210> 18
   <211> 27
   <212> DNA
   <213> INDIVIDUAL ISOLATE: Primer 2090
<400> 18
   catgacaaaa ttatttgatt taatcac 27
<210> 19
   <211> 22
   <212> DNA
   <213> INDIVIDUAL ISOLATE: Oligonukleotid 2091parS
<400> 19
   gatcctgttc cacgtgaaac ag 22
<210> 20
   <211> 22
   <212> DNA
   <213> INDIVIDUAL ISOLATE: Oligonucleotid 2092parS
<400> 20
   gatcctgttt cacgtggaac ag 22

## Patentansprüche

1. Verfahren zur selektiven Einführung einer Doppelbindung in ein Steroidgerüst durch Überexpression von Dehydrogenasen, **dadurch gekennzeichnet, daß** man
a) ein Dehydrogenasegen aus einem Bakterium Bacillus sphaericus isoliert, kloniert und amplifiziert.
b) Promotor- und Terminator-Elemente des Dehydrogenasegens oder andere Promotor- und Terminator-Elemente aus dem gleichen oder einem weiteren Bakterium isoliert, kloniert und amplifiziert
c) Expressionsplasmide konstruiert, worin das Dehydrogenasegen aus a), flankiert von Promotor- und Terminatorsequenz des Dehydrogenasegens oder von anderen Promotor- und Terminator-Elementen aus b), enthalten ist.
d) mit dem unter c) hergestellten Expressionsplasmid Bakterien transformiert und
e) die so hergestellten Bakterien kultiviert und mit diesen Kulturen die selektive Dehydrierung im Steroidgerüst durchführt, wobei
i) eine hohe Substratkonzentration bei unveränderter Laufzeit eingesetzt wird und
ii) keine störenden Nebenzonen entstehen.

2. Verfahren zur selektiven Einführung einer Doppelbindung in ein Steroidgerüst durch Überexpression von A'-Dehydrogenasen gemäß Anspruch 1, **dadurch gekennzeichnet, daß** man
a) ein Δ¹-Dehydrogenasegen aus einem Bakterium Bacillus sphaericus isoliert, kloniert und amplifiziert,
b) Promotor- und Terminator-Elemente des Δ¹-Dehydrogenasegens oder andere Promotor- und Terminator-Elemente aus dem gleichen oder einem weiteren Bakterium isoliert, kloniert und amplifiziert
c) Expressionsplasmide konstruiert, worin das Δ¹-Dehydrogenasegen aus a), flankiert von Promotor- und Terminatorsequenz des Δ¹-Dehydrogenasegens oder von anderen Promotor- und Terminator-Elementen aus b), enthalten ist,
d) mit dem unter c) hergestellten Expressionsplasmid Bakterien transformiert und
e) die so hergestellten Bakterien kultiviert und mit diesen Kulturen die selektive Dehydrierung im Steroidgerüst durchführt, wobei
i) eine hohe Substratkonzentration bei unveränderter Laufzeit eingesetzt wird und
ii) keine störenden Nebenzonen entstehen.

3. Verfahren zur selektiven Einführung einer Doppelbindung in ein Steroidgerüst durch Überexpression von 3-Ketosteroid-Δ¹-Dehydrogenasen gemäß Anspruch 2, **dadurch gekennzeichnet, daß** man
a) das 3-Ketosteroid-Δ¹-Dehydrogenasegen aus einem Bakterium Bacillus sphaericus isoliert, kloniert und amplifiziert,
b) Promotor- und Terminator-Elemente des 3-Ketosteroid-Δ¹-Dehydrogenasegens oder andere Promotor- und Terminator-Elemente aus dem gleichen oder einem weiteren Bakterium isoliert, kloniert und amplifiziert,
c) Expressionsplasmide konstruiert, worin das 3-Ketosteroid-Δ¹-Dehydrogenasegen aus a), flankiert von Promotor und Terminatorsequenz des 3-Ketosteroid-Δ¹-Dehydrogenasegens oder von anderen Promotor- und Terminator-Elementen aus b), enthalten ist,
d) mit dem unter c) hergestellten Expressionsplasmid Bakterien transformiert und
e) die so hergestellten Bakterien kultiviert und mit diesen Kulturen die selektive Dehydrierung an Position 1 im Steroidgerüst durchführt, wobei
i) eine hohe Substratkonzentration bei unveränderter Laufzeit eingesetzt wird und
ii) keine störenden Nebenzonen entstehen.

4. Verfahren nach einem der Ansprüche 1 bis 3, **dadurch gekennzeichnet, daß** die unter b) und d) genannten Bakterien zur *gram-*positiven Gattung *Bacillus* und *Arthrobacter* und zur gram-negativen Gattung *Escherichia coli* und *Pseudomonas* gehören.

5. Verfahren nach Anspruch 4, **dadurch gekennzeichnet, daß** die Bakterien zur Spezies *Bacillus* spec., *Bacillus subtilis, Bacillus sphaericus, Bacillus megaterium, Bacillus licheniformis, Bacillus lentus, Arthrobacter simplex, Brevibacterium maris* und *Pseudomonas species* gehören.

6. 3-Ketosteroid-Δ¹-Dehydrogenasegen aus *Bacillus sphaericus* gemäß Seq. ID No. 10.

7. 3-Ketosteroid-Δ¹-Dehydrogenase aus *Bacillus sphaericus* gemäß Seq. ID No. 11.

8. 3-Ketosteroid-Δ¹-Dehydrogenasegen Promotor aus *Bacillus sphaericus* gemäß Seq. ID No. 9.

9. Plasmide, enthaltend mindestens eine DNA-Sequenz gemäß einem der Ansprüche 6 und 8.

10. Plasmide gemäß Anspruch 9, **dadurch gekennzeichnet, daß** diese geeignete Promotoren und Terminatoren zur Überexpression von Δ¹-Dehydrogenasen in Bakterien enthalten.

11. Plasmide gemäß Anspruch 10, **dadurch gekennzeichnet, daß** als Terminatoren der Terminator des 3-Ketosteroid-Δ¹-Dehydrogenasegens *von Bacillus sphaericus* gemäß Seq. ID No. 10, oder Terminatoren von *Escherichia coli* oder *Bacillus subtilis* und als Promotoren der Promotor des 3-Kelosteroid-Δ¹-Dehydrogenasegens *von Bacillus sphaericus* gemäß Seq. ID No. 9, oder konstitutive Promotoren, oder Promotoren der Bacteriophagen Φ29 und SPO1, oder induzierbare Promotoren aus *Bacillus subtilis* oder Hybridpromotoren verwendet werden.

12. Plasmide gemäß Anspruch 11, **dadurch gekennzeichnet, daß** als Terminator der *Escherichia coli* Terminator t*(rmB),* der *Bacillus subtilis* Terminator t(*senS*) oder t(*senN*) und als Promotoren der konstitutive Promotor p(veg), als induzierbarer Promotor p(*aprE*) oder p(*sacB*) aus *Bacillus subtilis* oder als Hybridpromotor ein *lacl* kontrollierter SPO1-Promotor verwendet werden.

13. Verwendung der Plasmide gemäß den Ansprüchen 9 bis 12, zur Transformation von Bakterien, die zur Überexpression von Δ¹-Dehydrogenasen befähigt sind.

14. DNA-Sequenzen gemäß Anspruch 6, die für ein Protein mit 3-Ketosteroid-Δ¹-Dehydrogenase-Aktivität kodiert, und deren DNA-Sequenzen eine Homologie von mehr als 80% im Vergleich mit dem Seq Id. No. 10 aufweisen.

15. DNA-Sequenzen gemäß dem Anspruch 6, die für ein Protein mit 3-Ketosteroid-Δ¹-Dehydrogenase-Akctivität kodiert, und deren DNA Sequenzen eine Homologie von mehr als 90% im Vergleich mit dem Seq Id. No. 10 aufweisen

16. DNA-Sequenzen gemäß Anspruch 6, die für ein Protein mit 3-Ketosteroid-Δ¹-Dehydrogenase-Aktivität kodiert, und deren DNA-Sequenzen eine Homologie von mehr als 95% im Vergleich mit dem Seq Id. No. 10 aufweisen.

17. Protein-Sequenzen, gemäß Anspruch 7, die eine Homologie von mindestens 90% im Vergleich zu dem Seq. Id. No. 11 aufweisen und eine 3-Ketosteroid-Δ¹-Dehydrogenase-Aktivität besitzen.

18. Protein-Sequenzen, gemäß Anspruch 7, die eine Homologie von mindestens 95% im Vergleich zu dem Seq. Id. No. 11 aufweisen und eine 3-Ketosteroid-Δ¹-Dehydrogenase-Aktivität besitzen.

19. Promotor DNA-Sequenz gemäß Anspruch 8, deren DNA-Sequenz eine Homologie von mehr als 80% im Vergleich zu dem Seq. Id. No. 9 aufweist.

20. Promotor DNA-Sequenz gemäß Anspruch 8, deren DNA-Sequenz eine Homologie von mehr als 90% im Vergleich zu dem Seq. Id. No. 9 aufweist.

21. Promotor DNA-Sequenz gemäß Anspruch 8, deren DNA-Sequenz eine Homologie von mehr als 95% im Vergleich zu dem Seq. Id. No. 9 aufweist.

22. *Bacillus sphaericus* 3-Ketosteroid-Δ¹-Dehydrogenase Oligonukleotide gemäß den Sequenzen Seq. ID No. 15, Seq. ID No. 16, Seq. ID No.17 und Seq. ID No. 18.

23. parS Oligonukleotide gemäß den Sequenzen Seq. ID No.19 und Seq. ID No. 20.

24. Verwendung der durch die DNA-Sequenzen gemäß Anspruch 6 kodierten Protein und der durch die DNA- Sequenzen gemäß den Ansprüchen 14 bis 16,kodierten Proteine zur selektiven Dehydrierung von Steroiden.

25. Verwendung der Proteine gemäß den Ansprüchen 7, 17 und 18 zur selektiven Dehydrierung von Steroiden.

26. Verwendung gemäß den Ansprüchen 24 und 25, **dadurch gekennzeichnet, daß** das dehydrierte Steroid Betamethason, Clobetason, Clocortoton, Δ¹-11β,17α-Dihydroxy-6α,9α-difluor-16α-methylrogesteron. 11β,21-Dihydroxy-2`-methyt-5'βH-pregn-4-eno[17,16-d]oxazol-3,20-dion, Deflazacort, Deflazacortalkohol, Dexamethason, Diflocortolon, Fluocinolonacetonid, Fluocortolon, Hydroxysäure und Prednisolon ist.

## Claims

1. Process for selective introduction of a double bond into a steroid skeleton by overexpression of dehydrogenases, **characterized in that**
a) a dehydrogenase gene is isolated from a Bacillus sphaericus bacterium, cloned and amplified,
b) promoter and terminator elements of the dehydrogenase gene or other promoter and terminator elements are isolated from the same or another bacterium, cloned and amplified,
c) expression plasmids are designed in which the dehydrogenase gene from a), flanked by promoter and terminator sequences of the dehydrogenase gene or by other promoter and terminator elements from b), is contained,
d) bacteria are transformed with the expression plasmid that is produced under c), and
e) the thus produced bacteria are cultivated, and the selective dehydrogenation in the steroid skeleton is performed with these cultures, whereby
i) a high substrate concentration at unaltered operating times is used, and
ii) no disruptive secondary zones are produced.

2. Process for selective introduction of a double bond into a steroid skeleton by overexpression of A'-dehydrogenases according to Claim 1, **characterized in that**
a) a Δ¹-dehydrogenase gene is isolated from a Bacillus sphaericus bacterium, cloned and amplified,
b) promoter and terminator elements of the Δ¹-dehydrogenase gene or other promoter and terminator elements are isolated from the same or another bacterium, cloned and amplified,
c) expression plasmids are designed, in which the Δ¹-dehydrogenase gene from a), flanked by promoter and terminator sequences of the Δ¹-dehydrogenase gene or by other promoter and terminator elements from b), is contained,
d) bacteria are transformed with the expression plasmid that is produced under c), and
e) the thus produced bacteria are cultivated, and the selective dehydrogenation in the steroid skeleton is performed with these cultures, whereby
i) a high substrate concentration at unaltered operating times is used, and
ii) no disruptive secondary zones are produced.

3. Process for selective introduction of a double bond into a steroid skeleton by
overexpression of 3-keto steroid-Δ¹-dehydrogenases according to Claim 2, **characterized in that**
a) the 3-keto steroid-Δ¹-dehydrogenase gene is isolated from a Bacillus sphaericus bacterium, cloned and amplified,
b) promoter and terminator elements of the 3-keto steroid-Δ¹-dehydrogenase gene or other promoter and terminator elements are isolated from the same or another bacterium, cloned and amplified,
c) expression plasmids are designed, in which the 3-keto steroid-Δ¹-dehydrogenase gene from a), flanked by promoter and terminator sequences of the 3-keto steroid-Δ¹-dehydrogenase gene or by other promoter and terminator elements from b), is contained,
d) bacteria are transformed with the expression plasmid that is produced under c), and
e) the thus produced bacteria are cultivated, and the selective dehydrogenation at 1-position in the steroid skeleton is performed with these cultures, whereby
i) a high substrate concentration at unaltered operating times is used, and
ii) no disruptive secondary zones are produced.

4. Process according to one of Claims 1 to 3, **characterized in that** the bacteria that are mentioned under b) and d) include the *gram-*positive genera *Bacillus* and *Arthrobacter* and the *gram*-negative genera *Escherichia coli* and *Pseudomonas.*

5. Process according to Claim 4, **characterized in that** the bacteria include the species *Bacillus spec., Bacillus subtilis, Bacillus sphaericus, Bacillus megaterium, Bacillus licheniformis, Bacillus lentus, Arthrobacter simplex, Brevibacterium maris* and *Pseudomonas species.*

6. 3-Keto steroid-Δ¹-dehydrogenase gene from *Bacillus sphaericus* according to Seq. ID No. 10.

7. 3-Keto steroid-Δ¹-dehydrogenase from *Bacillus sphaericus* according to Seq. ID No. 11.

8. 3-Keto steroid-Δ¹-dehydrogenase gene promoter from *Bacillus sphaericus* according to Seq. ID No. 9.

9. Plasmids that contain at least one DNA sequence according to one of Claims 6 and 8.

10. Plasmids according to Claim 9, **characterized in that** the latter contain suitable promoters and terminators for overexpression of Δ¹-dehydrogenases in bacteria.

11. Plasmids according to Claim 10, **characterized in that** the terminator of the 3-keto steroid-Δ¹-dehydrogenase gene of *Bacillus sphaericus* according to Seq. ID No. 10, or terminators of *Escherichia coli* or *Bacillus subtilis* are used as terminators, and the promoter of the 3-keto steroid-Δ¹-dehydrogenase gene of *Bacillus sphaericus* according to Seq. ID No. 9, or constitutive promoters, or promoters of the bacteriophages Φ29 and SPO1, or inducible promoters from *Bacillus subtilis* or hybrid promoters are used as promoters.

12. Plasmids according to Claim 11, **characterized in that** the *Escherichia coli* terminator t(*rrnB)*, the *Bacillus subtilis* terminator t(*senS)* or t(*senN*) is used as a terminator, and the constitutive promoter p*(veg)* is used as a promoter, p(*aprE*) or p(*sacB*) from *Bacillus subtilis* is used as an inducible promoter, or a *lacI*-controlled SPO1-promoter is used as a hybrid promoter.

13. Use of the plasmids according to Claims 9 to 12 for the transformation of bacteria that are capable of overexpression of A'-dehydrogenases.

14. DNA sequences according to Claim 6, which code for a protein with 3-keto steroid-Δ¹-dehydrogenase activity, and whose DNA sequences have a homology of more than 80% in comparison to Seq Id. No. 10.

15. DNA sequences according to Claim 6, which code for a protein with 3-keto steroid-Δ¹-dehydrogenase activity, and whose DNA sequences have a homology of more than 90% in comparison to Seq Id. No. 10.

16. DNA sequences according to Claim 6, which code for a protein with 3-keto steroid-Δ¹-dehydrogenase activity, and whose DNA sequences have a homology of more than 95% in comparison to Seq Id. No. 10.

17. Protein sequences according to Claim 7, which have a homology of at least 90% in comparison to Seq. Id. No. 11 and have a 3-keto steroid-Δ¹-dehydrogenase activity.

18. Protein sequences according to Claim 7, which have a homology of at least 95% in comparison to Seq. Id. No. 11 and have a 3-keto steroid-Δ¹-dehydrogenase activity.

19. DNA-sequence promoter according to Claim 8, whose DNA sequence has a homology of more than 80% in comparison to Seq. Id. No. 9.

20. DNA-sequence promoter according to Claim 8, whose DNA sequence has a homology of more than 90% in comparison to Seq. Id. No. 9.

21. DNA-sequence promoter according to Claim 8, whose DNA sequence has a homology of more than 95% in comparison to Seq. Id. No. 9.

22. *Bacillus sphaericus* 3-keto steroid-Δ¹-dehydrogenase oligonucleotides according to the sequences Seq. ID No. 15, Seq. ID No. 16, Seq. ID No. 17 and Seq. ID No. 18.

23. *parS* Oligonucleotides according to the sequences Seq. ID No. 19 and Seq. ID No. 20.

24. Use of the proteins that are coded by the DNA sequences according to Claim 6 and of the proteins that are coded by the DNA sequences according to Claims 14 to 16 for selective dehydrogenation of steroids.

25. Use of the proteins according to Claims 7, 17 and 18 for selective dehydrogenation of steroids.

26. Use according to Claims 24 and 25, **characterized in that** the dehydrogenated steroid is betamethasone, clobetasone, clocortolone, Δ¹-11β, 17α-dihydroxy-6α,9α-difluoro-16α-methylprogesterone, 11β,21-dihydroxy-2'-methyl-5'βH-pregn-4-eno[17,16-d]oxazole-3,20-dione, deflazacort, deflazacort alcohol, dexamethasone, diflocortolone, fluocinolone acetonide, fluocortolone, hydroxy acid and prednisolone.

## Revendications

1. Procédé pour l'introduction sélective d'une double liaison dans une structure stéroïde par surexpression de déshydrogénases, **caractérisé en ce qu'**on
a) isole un gène de déshydrogénase d'une bactérie Bacillus sphaericus, on le clone et on l'amplifie,
b) isole des éléments de type promoteur et de type terminateur du gène de déshydrogénase ou d'autres éléments de type promoteur et terminateur de la même bactérie ou d'une autre bactérie, on les clone et on les amplifie,
c) construit des plasmides d'expression, dans lesquels est contenu le gène de déshydrogénase de a), flanqué de la séquence de promoteur et de terminateur du gène de déshydrogénase ou d'autres éléments promoteurs et terminateurs de b),
d) transforme des bactéries avec le plasmide d'expression préparé au point c) et
e) cultive les bactéries ainsi préparées et on réalise avec ces cultures la déshydrogénation sélective dans la structure stéroïde,
i) en utilisant une concentration élevée en substrat avec un temps de fonctionnement inchangé et
ii) sans formation de zones secondaires gênantes.

2. Procédé pour l'introduction sélective d'une double liaison dans une structure stéroïde par surexpression de Δ¹-déshydrogénases selon la revendication 1, **caractérisé en ce qu'**on
a) isole un gène de Δ¹-déshydrogénase d'une bactérie Bacillus sphaericus, on le clone et on l'amplifie,
b) isole des éléments de type promoteur et de type terminateur du gène de Δ¹-déshydrogénase ou d'autres éléments de type promoteur et terminateur de la même bactérie ou d'une autre bactérie, on les clone et on les amplifie,
c) construit des plasmides d'expression, dans lesquels est contenu le gène de Δ¹-déshydrogénase de a), flanqué de la séquence de promoteur et de type terminateur du gène de Δ¹-déshydrogénase ou d'autres éléments de type promoteur et de type terminateur de b),
d) transforme des bactéries avec le plasmide d'expression préparé au point c) et
e) cultive les bactéries ainsi préparées et on réalise avec ces cultures la déshydrogénation sélective dans la structure stéroïde,
i) en utilisant une concentration élevée en substrat avec un temps de fonctionnement inchangé et
ii) sans formation de zones secondaires gênantes.

3. Procédé pour l'introduction sélective d'une double liaison dans une structure stéroïde par surexpression de 3-cétostéroïde-Δ¹-déshydrogénases selon la revendication 2, **caractérisé en ce qu'**on
a) isole un gène de 3-cétostéroïde-Δ¹-déshydrogénase d'une bactérie Bacillus sphaericus, on le clone et on l'amplifie,
b) isole des éléments de type promoteur et de type terminateur du gène de 3-cétostéroïde-Δ¹-déshydrogénase ou d'autres éléments de type promoteur et de type terminateur de la même bactérie ou d'une autre bactérie, on les clone et on les amplifie,
c) construit des plasmides d'expression, dans lesquels est contenu le gène de 3-cétostéroïde-Δ¹-déshydrogénase de a), flanqué de la séquence de promoteur et de terminateur du gène de 3-cétostéroïde-Δ¹-déshydrogénase ou d'autres éléments de type promoteur et de type terminateur de b),
d) transforme des bactéries avec le plasmide d'expression préparé au point c) et
e) cultive les bactéries ainsi préparées et on réalise avec ces cultures la déshydrogénation sélective en position 1 dans la structure stéroïde,
i) en utilisant une concentration élevée en substrat avec un temps de fonctionnement inchangé et
ii) sans formation de zones secondaires gênantes.

4. Procédé selon l'une quelconque des revendications 1 à 3, **caractérisé en ce que** les bactéries mentionnées aux points b) et d) appartiennent aux genres gram-positifs Bacillus et Arthrobacter et aux genres gram-négatifs Escherichia coli et Pseudomonas.

5. Procédé selon la revendication 4, **caractérisé en ce que** les bactéries appartiennent aux espèces Bacillus spec., Bacillus subtilis, Bacillus sphaericus, Bacillus megaterium, Bacillus licheniformis, Bacillus lentus, Arthrobacter simplex, Brevibacterium maris et aux espèces Pseudomonas.

6. Gène de 3-cétostéroïde-Δ¹-déshydrogénase de Bacillus sphaericus selon la SEQ. ID. No. 10.

7. 3-cétostéroïde-Δ¹-déshydrogénase de Bacillus sphaericus selon la SEQ. ID. No. 11.

8. Promoteur du gène de 3-cétostéroïde-Δ¹-déshydrogénase de Bacillus sphaericus selon la SEQ. ID. No. 9.

9. Plasmides, contenant au moins une séquence d'ADN selon l'une quelconque des revendications 6 et 8.

10. Plasmides selon la revendication 9,
**caractérisés en ce qu'**ils contiennent des promoteurs et des terminateurs appropriés pour la surexpression de Δ¹-déshydrogénases dans les bactéries.

11. Plasmides selon la revendication 10,
**caractérisés en ce qu'**on utilise comme terminateurs le terminateur du gène de 3-cétostéroïde-Δ¹-déshydrogénase de Bacillus sphaericus selon la SEQ. ID. No. 10, ou des terminateurs d'Escherichia coli ou de Bacillus subtilis et comme promoteurs le promoteur du gène de 3-cétostéroïde-Δ¹-déshydrogénase de Bacillus sphaericus selon la SEQ. ID. No. 9, ou des promoteurs constitutifs
ou des promoteurs des bactériophages ϕ29 et SPO1, ou des promoteurs pouvant être induits de Bacillus subtilis ou des promoteurs hybrides.

12. Plasmides selon la revendication 11,
**caractérisés en ce qu'**on utilise comme terminateur le terminateur d'Escherichia coli t(rmB), le terminateur de Bacillus subtilis t (senS) ou t(senN) et comme promoteur le promoteur constitutif p(veg), comme promoteur pouvant être induit le promoteur p(aprE) ou p(sacB) de Bacillus subtilis ou comme promoteur hybride un promoteur SPO1 contrôlé par lacl.

13. Utilisation des plasmides selon les revendications 9 à 12, pour la transformation de bactéries qui sont aptes à la surexpression de Δ¹-déshydrogénases.

14. Séquences d'ADN selon la revendication 6, qui codent pour une protéine avec une activité 3-cétostéroïde-Δ¹-déshydrogénase, et dont les séquences d'ADN présentent une homologie supérieure à 80% par rapport à la SEQ. ID. No. 10.

15. Séquences d'ADN selon la revendication 6, qui codent pour une protéine avec une activité 3-cétostéroïde-Δ¹-déshydrogénase, et dont les séquences d'ADN présentent une homologie supérieure à 90% par rapport à la SEQ. ID. No. 10.

16. Séquences d'ADN selon la revendication 6, qui codent pour une protéine avec une activité 3-cétostéroïde-Δ¹-déshydrogénase, et dont les séquences d'ADN présentent une homologie supérieure à 95% par rapport à la SEQ. ID. No. 10.

17. Séquences de protéine selon la revendication 7, qui présentent une homologie d'au moins 90% par rapport à la SEQ. ID. No. 11 et qui possèdent une activité de 3-cétostéroïde-Δ¹-déshydrogénase.

18. Séquences de protéine selon la revendication 7, qui présentent une homologie d'au moins 95% par rapport à la SEQ. ID. No. 11 et qui possèdent une activité de 3-cétostéroïde-Δ¹-déshydrogénase.

19. Séquence d'ADN de promoteur selon la revendication 8, dont la séquence d'ADN présente une homologie supérieure à 80% par rapport à la SEQ. ID. No. 9.

20. Séquence d'ADN de promoteur selon la revendication 8, dont la séquence d'ADN présente une homologie supérieure à 90% par rapport à la SEQ. ID. No. 9.

21. Séquence d'ADN de promoteur selon la revendication 8, dont la séquence d'ADN présente une homologie supérieure à 95% par rapport à la SEQ. ID. No. 9.

22. Oligonucléotide de 3-cétostéroïde-Δ¹-déshydrogénase de Bacillus sphaericus selon les séquences SEQ. ID. No. 15, SEQ. ID. No. 16, SEQ. ID. No. 17 et SEQ. ID. No. 18.

23. Oligonucléotide parS selon les séquences SEQ. ID. No. 19 et SEQ. ID. No. 20.

24. Utilisation des protéines codées par les séquences d'ADN selon la revendication 6 et des protéines codées par les séquences d'ADN selon les revendications 14 à 16, pour la déshydratation sélective de stéroïdes.

25. Utilisation des protéines selon les revendications 7, 17 et 18 pour la déshydrogénation sélective de stéroïdes.

26. Utilisation selon les revendications 24 et 25, **caractérisée en ce que** le stéroïde déshydrogéné est le Betamethason, le Clobetason, le Clocortoton, la Δ¹-11β,17α-dihydroxy-6α,9α-difluoro-16α-méthylprogestérone, la 11β,21-dihydroxy-2'-méthyl-5'βH-pregn-4-éno[17,16-d]oxazol-3,20-dione, le Deflazacort, l'alcool de Deflazacort, le Dexamethason, le Diflocortolon, le Fluocinolonacetonid, le Fluocortolon, l'hydroxyacide et le Prednisolon.
